# EUROPÄISCHE PATENTSCHRIFT

(11) **EP 1 045 832 B1**
(45) Veröffentlichungstag und Bekanntmachung des Hinweises auf die Patenterteilung: **23.03.2005**
(21) Anmeldenummer: 98962422.6
(22) Anmeldetag: 08.12.1998
(51) Int. Cl.: C07C 271/56, C07C 271/24, C07C 333/24, C07C 333/06, A23K 1/16, A61K 31/325

(54) **URETHANE, IHRE THIO- UND DITHIOANALOGA, DEREN SALZE, DIESE VERBINDUNGEN ENTHALTENDE ARZNEIMITTEL UND DEREN VERWENDUNG SOWIE VERFAHREN ZU IHRER HERSTELLUNG**
NOVEL URETHANES, THIO AND DITHIO ANALOGUES, THE SALTS THEREOF, MEDICAMENTS CONTAINING SAID COMPOUNDS, USE AND METHOD FOR THE PRODUCTION THEREOF
NOUVEAUX URETHANES, LEURS ANALOGUES THIO ET DITHIO, LEURS SELS, MEDICAMENTS CONTENANT CES COMPOSES, LEUR UTILISATION ET LEUR PROCEDE DE PRODUCTION

(30) Priorität: 10.12.1997 DE 19754795
(43) Veröffentlichungstag der Anmeldung: 25.10.2000
(73) Patentinhaber: Boehringer Ingelheim Pharma GmbH & Co.KG, 55218 Ingelheim am Rhein (DE)
(72) Erfinder: MAIER, Roland, D-88400 Biberach (DE); MÜLLER, Peter, Stamford, CT 06903 (US); ADELGOSS, Gebhard, D-88400 Biberach (DE); SCHILCHER, Gebhard, D-88441 Mittelbiberach (DE); HURNAUS, Rudolf, D-88400 Biberach (DE); MARK, Michael, D-88400 Biberach (DE); EISELE, Bernhard, D-88400 Biberach (DE)
(74) Vertreter: Kläs, Heinz-Gerd, Dr.
(86) Internationale Anmeldenummer: PCT/EP1998/007962
(87) Internationale Veröffentlichungsnummer: WO 1999/029662

(56) Entgegenhaltungen:
- EP-A- 0 596 326
- WO-A-95/29148
- DE-A- 4 239 151
- DE-A- 4 438 020

## Beschreibung

Die vorliegende Erfindung betrifft neue Urethane, ihre Thiound Dithioanaloga, deren Salze mit physiologisch verträglichen organischen und anorganischen Säuren, Verfahren zur Herstellung dieser Verbindungen und diese enthaltende Arzneimittel.

Die erfindungsgemäßen Verbindungen stellen Inhibitoren der Cholesterolbiosynthese dar, insbesondere Inhibitoren des Enzyms 2,3-Epoxisqualen-Lanosterol-Cyclase, eines Schlüsselenzyms der Cholesterolbiosynthese. Die erfindungsgemäßen Verbindungen sind geeignet zur Behandlung und Prophylaxe von Hyperlipidämien, Hypercholesterolämien und der Atherosklerose. Weitere mögliche Anwendungsgebiete ergeben sich für die Behandlung von hyperproliferativen Haut- und Gefässerkrankungen, Tumoren, Gallensteinleiden sowie von Mykosen.

Verbindungen,die in die Cholesterolbiosynthese eingreifen, sind für die Behandlung einer Reihe von Krankheitsbildern von Bedeutung. Hier sind vor allem Hypercholesterolämien und Hyperlipidämien zu nennen, die Risikofaktoren für das Entstehen atherosklerotischer Gefäßveränderungen und ihrer Folgeerkrankungen wie beispielsweise koronare Herzkrankheit, cerebrale Ischämie, Claudicatio intermittens und Gangrän darstellen.

Die Bedeutung überhöhter Serum-Cholesterol-Spiegel als Hauptrisikofaktor für das Entstehen atherosklerotischer Gefäßveränderungen wird allgemein anerkannt. Umfangreiche klinische Studien haben zu der Erkenntnis geführt, daß durch Erniedrigung des Serumcholesterols das Risiko, an koronaren Herzkrankheiten zu erkranken, verkleinert werden kann (Current Opinion in Lipidology 2(4), 234 [1991]; Exp. Opin. Ther. Patents 7(5), 441-455 [1997]). Da der größte Teil des Cholesterols im Organismus selbst synthetisiert und nur ein geringer Teil mit der Nahrung aufgenommen wird, stellt die Hemmung der Biosynthese einen besonders attraktiven Weg dar, den erhöhten Cholesterolspiegel zu senken.

Daneben werden als weitere mögliche Anwendungsgebiete von Cholesterolbiosynthesehemmern die Behandlung hyperproliferativer Haut- und Gefäßerkrankungen sowie von Tumorerkrankungen, die Behandlung und Prophylaxe von Gallensteinleiden sowie der Einsatz bei Mykosen beschrieben. Hierbei handelt es sich im letzten Fall um einen Eingriff in die Ergosterolbiosynthese in Pilzorganismen, welche weitgehend analog der Cholesterolbiosynthese in Säugerzellen verläuft.

Weiterhin offenbart die DE-A-4239 151 N,N-disubstituierte Arylcycloalkylamine, die eine hemmende Wirkung auf die Biosynthese von Cholesterol, insbesondere eine hemmende Wirkung auf eines der Schlüsselenzyme der Cholesterolbiosynthese, die 2,3-Epoxisqualen-Lanosterol-Cyclase, aufweisen.

Die Cholesterol- bzw. die Ergosterolbiosynthese verläuft, ausgehend von Essigsäure, über eine größere Zahl von Reaktionsschritten. Dieser Vielstufenprozeß bietet eine Reihe von Eingriffsmöglichten, von denen als Beispiele genannt seien:

Für die Inhibition des Enzyms 3-Hydroxy-3-methylglutaryl-coenzym A (HMG-CoA)-Synthase werden β-Lactone- und β-Lactame mit potentieller antihypercholesterolämischer Wirkung erwähnt (siehe J. Antibiotics 40, 1356 [1987], US-A-4,751,237, EP-A-0 462 667, US-A-4,983,597).

Beispiele für Inhibitoren des Enzyms HMG-CoA-Reduktase stellen 3,5-Dihydroxycarbonsäuren vom Mevinolintyp und deren δ-Lactone dar, deren Vertreter Lovastatin, Simvastatin, Pravastatin, Fluvastatin, Atorvastatin sowie Cerivastatin in der Therapie von Hypercholesterolämien Verwendung finden. Weitere mögliche Anwendungsgebiete dieser Verbindungen sind Pilzinfektionen (US-A-4,375,475, EP-A-0 113 881, US-A-5,106,992), Hauterkrankungen (EP-A-0 369 263) sowie Gallensteinleiden und Tumorerkrankungen (US-A-5,106,992; Lancet 339, 1154-1156 [1992]).

Die Hemmung der Proliferation glatter Muskelzellen durch Lovastatin ist beschrieben in Cardiovasc. Drugs. Ther. 5, Suppl. 3, 354 [1991]. Tocotrienol, ein ungesättigtes Analoges des Vitamin E, und dessen Analoga stellen eine weitere Substanzklasse dar, die auf die HMG-CoA-Reduktase einwirkt (Exp. Opin. Ther. Patents 7 (5), 446 [1997]).

Inhibitoren des Enzyms Squalen-Synthetase sind z.B. Isoprenoid -(phosphinylmethyl)-phosphonate, deren Eignung zur Behandlung von Hypercholesterolämien, Gallensteinleiden und Tumorerkrankungen in EP-A-0 409 181 sowie in J. Med. Chemistry 34, 1912 [1991] beschrieben ist, ferner α-Phosphonosulfinat-Verbindungen (EP-A-0 698 609), die Verbindungen J-104,118 und J-104,123 (Tetrahedron 52, 13881-13894, [1996]) sowie Cyclobutanderivate (WO 96/33159). Ein Überblick über Squalen-Synthethase-Inhibitoren findet sich in Exp. Opin. Ther. Patents 7 (5), 446-448 [1997].

Als Inhibitoren des Enzyms Squalen-Epoxidase sind bekannt Allylamine wie Naftidin und Terbinafin, die als Mittel gegen Pilzerkrankungen Eingang in die Therapie gefunden haben, sowie das Allylamin NB-598 mit antihypercholesterolämischer Wirkung (J. Biol. Chemistry 265, 18075-18078 (1990]) und Fluorsqualen-Derivate mit hypocholesterolämischer Wirkung (US-A-5,011,859). Des weiteren sind Piperidine und Azadecaline mit potentieller hypocholesterolämischer und/oder antifungaler Wirkung beschrieben, deren Wirkmechanismus nicht eindeutig geklärt ist und welche Squalenepoxidase- und/oder 2,3-Epoxisqualen-Lanosterol-Cyclase-Inhibitoren darstellen (EP-A-0 420 116, EP-A-0 468 434, US-A-5,084,461 und EP-A-0 468 457). Weitere Vertreter sind beschrieben in Exp. Opin. Ther. Patents 7 (5), 448-449 [1997].

Beispiele für Inhibitoren des Enzyms 2,3-Epoxisqualen-Lanosterol-Cyclase sind Diphenylderivative (EP-A-0 464 465), Aminoalkoxybenzol-Derivate (EP-A-0 410 359, J. Lipid. Res. 38, 373-390, [1997]) sowie Piperidin-Derivate (J. Org. Chem. 57, 2794-2903 [1992], die eine antifungale Wirkung besitzen. Des weiteren wird dieses Enzym in Säugetierzellen durch Decaline, Azadecaline und Indanderivate (WO 89/08450; J. Biol. Chemistry 254, 11258-11263 [1981]; Biochem. Pharmacology 37, 1955-1964 [1988] und J 64 003 144), ferner durch 2-Aza-2,3-dihydrosqualen und 2,3-Epiminosqualen (Biochem. Pharmacology 34, 2765-2777 [1985]), durch Squalenoid-Epoxid-Vinylether (J. Chem. Soc. Perkin Trans. I, 461 [1988]) und 29-Methyliden-2,3-oxidosqualen (J. Amer. Chem. Soc. 113, 9673-9674 [1991]) inhibiert. Weitere Beispiele sind Pyridin- bzw. Pyrimidin-Derivate (WO 97/06802), heterobicyclische Alkylamine (WO 96/11201), Imidazolderivate (EP-A-0 757 988) sowie Isochinolinderivate (J. Med. Chemistry 39, 2302-2312, [1996]). Des weiteren sind beschrieben Harnstoffe (DE-A-4 438 021), Oxime (DE-A-4 412 692), eine Reihe von Amiden (DE-A-4 407 134, DE-A-4 407 135, DE-A-4 407 136, DE-A-4 407 138, DE-A-4 407 139, DE-A-4 412 691, DE-A-4 437 999, DE-A-4 438 000, DE-A-4 438 020, DE-A-4 438 082, DE-A-4 438 029, DE-A-4 438 054, DE-A-4 438 055, DE-A-4 438 082, DE-A-4 438 083, EP-A-0 599 203, EP-A-0 596 326) sowie Ester (WO 95/29148). Weitere Beispiele sind beschrieben in Exp. Opin. Ther. Patents 7(5), 448-449 [1997].

Schließlich sind als Inhibitoren des Enzyms Lanosterol-14α-Demethylase noch Steroidderivate mit potentieller antihyperlipidämischer Wirkung zu nennen, die gleichzeitig das Enzym HMG-CoA-Reduktase beeinflussen (US-A-5,041,431; J.Biol. Chemistry 266, 20070-20078 [1991]; US-A-5,034,548). Außerdem wird dieses Enzym durch die Antimykotika vom Azol-Typ inhibiert, welche N-substituierte Imidazole und Triazole darstellen. Zu dieser Klasse gehören beispielsweise die auf dem Markt befindlichen Antimykotika Ketoconazol und Fluconazol.

Die Verbindungen der nachfolgenden allgemeinen Formel I sind neu. Es wurde überraschenderweise gefunden, daß sie sehr wirksame Inhibitoren des Enzyms 2,3-Epoxisqualen-Lanosterol-Cyclase (Internationale Klassifizierung: EC 5.4.99.7) darstellen.

Das Enzym 2,3-Epoxisqualen-Lanosterol-Cyclase katalysiert einen Schlüsselschritt der Cholesterol- bzw. Ergosterol-Biosynthese, nämlich die Umwandlung des 2,3-Epoxisqualens in das Lanosterol, die erste Verbindung mit Steroidstruktur in der Biosynthesekaskade. Inhibitoren dieses Enzyms lassen gegenüber Inhibitoren früherer Biosyntheseschritte, wie beispielsweise HMG-CoA-Synthase und HMG-CoA-Reduktase, den Vorteil der höheren Selektivität erwarten, da die Inhibierung dieser frühen Biosyntheseschritte zur Abnahme biosynthetisch gebildeter Mevalonsäure führt und dadurch auch die Biosynthese der mevalonsäureabhängigen Substanzen Dolichol, Ubichinon und Isopentenyl-t-RNA negativ beeinflussen kann (vgl. J. Biol. Chemistry 265, 18075-18078 [1990].

Bei Inhibierung von Biosyntheseschritten nach der Umwandlung von 2,3-Epoxisqualen in Lanosterol besteht die Gefahr der Anhäufung von Intermediärprodukten mit Steroidstruktur im Organismus und der Auslösung dadurch bedingter toxischer Effekte. Dies ist beispielsweise für Triparanol, einem Desmosterol-Reduktase-Inhibitor, beschrieben. Diese Substanz mußte wegen Bildung von Katarakten, Ichthyosis und Alopecie vom Markt genommen werden (zitiert in J. Biol. Chemistry 265, 18075-18078 [1990]).

Wie bereits eingangs dargelegt sind Inhibitoren der 2,3-Epoxisqualen-Lanosterol-Cyclase bereits in der Literatur beschrieben. Es sind jedoch keinerlei Urethane sowie deren Thiooder Dithioanaloge als Inhibitoren der 2,3-Epoxisqualen-Lanosterol-Cyclase bekannt.

Die Erfindung betrifft die Bereitstellung von antihypercholesterolämischen Substanzen, die zur Behandlung und Prophylaxe der Atherosklerose geeignet sind und im Vergleich zu bekannten Wirkstoffen durch eine bessere antihypercholesterolämische Wirkung bei erhöhter Selektivität und damit erhöhter Sicherheit ausgezeichnet sind. Da die erfindungsgemäßen Verbindungen auf Grund ihrer hohen Wirksamkeit als Inhibitoren des Enzyms 2,3-Epoxisqualen-Lanosterol-Cyclase auch die Ergosterol-Biosynthese im Pilzorganismus inhibieren können, sind sie auch zur Behandlung von Mykosen geeignet.

Die vorliegende Erfindung betrifft die neuen Urethane sowie deren Thio- und Dithioanalogen der allgemeinen Formel 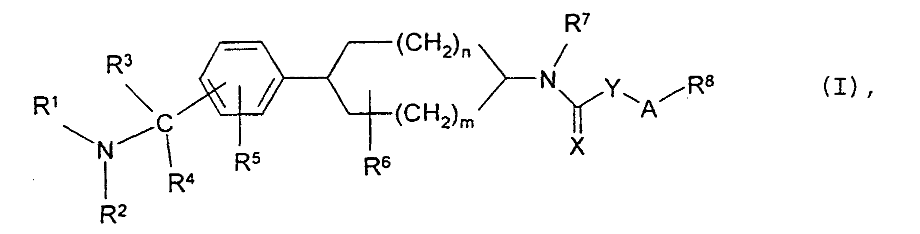 in der
m die Zahlen 0 oder 1,
n die Zahlen 1 oder 2,
A eine Einfachbindung, eine geradkettige oder verzweigte C₁₋₈-Alkylengruppe, eine C₂₋₈-Alkenylen- oder C₂₋₈-Alkinylengruppe, wobei eine ungesättigte Gruppe nicht direkt an den Rest Y gebunden ist,
X ein Sauerstoff- oder Schwefelatom,
Y ein Sauerstoff- oder Schwefelatom,
R¹ eine geradkettige oder verzweigte C₁₋₈-Alkylgruppe, eine C₁₋₆-Alkenylgruppe oder eine C₁₋₆-Alkinylgruppe, wobei die Mehrfachbindung von der Stickstoff-Kohlenstoff-Bindung isoliert ist,
R² ein Wasserstoffatom, eine geradkettige oder verzweigte C₁₋₈-Alkylgruppe, die durch eine Hydroxy- oder Alkoxygruppe substituiert sein kann, wobei ein Hydroxy- und Alkoxy-Substituent nicht in 1-Stellung gebunden ist, eine C₁₋₆-Alkenylgruppe oder eine C₁₋₆-Alkinylgruppe, wobei eine Mehrfachbindung von der Stickstoff-Kohlenstoff-Bindung isoliert ist, oder
R¹ und R² zusammen mit dem Stickstoffatom einen 5- bis 7-gliedrigen, gesättigten heterocyclischen Ring, in dem eine von dem Stickstoffatom isolierte Methylengruppe durch ein Sauerstoffoder Schwefelatom, durch eine -NH- oder -N(Alkyl)- Gruppe ersetzt sein kann,
R³ bis R⁶, die gleich oder verschieden sein können, Wasserstoffatome oder Alkylgruppen,
R⁷ eine geradkettige oder verzweigte C₁₋₆-Alkylgruppe, eine C₁₋₆-Alkenylgruppe oder eine C₁₋₆-Alkinylgruppe, wobei die Mehrfachbindung von der Stickstoff-Kohlenstoff-Bindung isoliert ist,
R⁸ eine C₃₋₇-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe oder, sofern A keine Einfachbindung darstellt, auch ein Wasserstoffatom bedeuten,
wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
mit der Maßgabe, daß trans-N-tert.Butoxycarbonyl-N-methyl-4-[4-(3-hydroxypropyl)methylaminomethylphenyl]cyclohexylamin ausgenommen wird, deren Enantiomere, Diastereomere, deren Gemische und deren Salze, insbesondere deren physiologisch verträglichen Säureadditionssalze.

Bevorzugt sind Verbindungen der allgemeinen Formel I, in der
m die Zahl 1,
n die Zahl 1,
A eine Einfachbindung, eine geradkettige oder verzweigte C₁₋₆-Alkylengruppe,
X ein Sauerstoff- oder Schwefelatom,
Y ein Sauerstoff- oder Schwefelatom,
R¹ eine geradkettige oder verzweigte C₁₋₆-Alkylgruppe,
R² ein Wasserstoffatom, eine geradkettige oder verzweigte C₁₋₆-Alkylgruppe, die durch eine Hydroxygruppe substituiert sein kann, wobei die Hydroxygruppe nicht in 1-Stellung gebunden ist, eineC₁₋₄-Alkenylgruppe oder eine C₁₋₄-Alkinylgruppe, wobei die Mehrfachbindung von der Stickstoff-Kohlenstoff-Bindung isoliert ist, oder
R¹ und R² zusammen mit dem Stickstoffatom einen 5-bis 7-gliedrigen, gesättigten heterocyclischen Ring, in dem eine von dem Stickstoffatom isolierte Methylengruppe durch ein Sauerstoffatom ersetzt sein kann,
R³ bis R⁶, die gleich oder verschieden sein können, Wasserstoffatome oder Methylgruppen,
R⁷ eine geradkettige oder verzweigte C₁₋₆-Alkylgruppe oder eine C₁₋₄-Alkenylgruppe, wobei die Mehrfachbindung von der Stickstoff-Kohlenstoff-Bindung isoliert ist,
R⁸ eine C₃₋₆-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe oder, sofern A keine Einfachbindung darstellt, auch ein Wasserstoffatom bedeuten,
wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
mit der Maßgabe, daß trans-N-tert.Butoxycarbonyl-N-methyl-4-[4-(3-hydroxypropyl)methylaminomethylphenyl]cyclohexylamin ausgenommen wird, deren Enantiomere, Diastereomere, deren Gemische und deren Salze, insbesondere deren physiologisch verträglichen Säureadditionssalze.

Besonders bevorzugt sind die Verbindungen der allgemeinen Formel I, in der
m die Zahl 1,
n die Zahl. 1,
A eine Einfachbindung oder eine Methylengruppe,
X ein Sauerstoff- oder Schwefelatom,
Y ein Sauerstoff- oder Schwefelatom,
R¹ eine Methyl- oder Ethylgruppe,
R² eine Methyl-, Ethyl-, Allyl- oder Propargylgruppe oder
R¹ und R² zusammen mit dem Stickstoffatom einen Pyrrolidin- oder Piperidinring,
R³ bis R⁶ Wasserstoffatome,
R⁷ eine Methylgruppe,
R⁸ eine gegebenenfalls durch ein Chlor- oder Fluoratom oder durch eine Methylgruppe substituierte Phenylgruppe bedeuten, deren Gemische und deren Salze, insbesondere deren physiologisch verträglichen Säureadditionssalze, insbesondere jedoch die Verbindungen
(1) trans-S-(4-Chlorphenyl)-N-4-[4-(dimethylaminomethyl)-phenyl]cyclohexyl-N-methyldithiocarbamat,
(2) trans-S-(3-Fluorbenzyl)-N-4-[4-(dimethylaminomethyl)-phenyl]cyclohexyl-N-methyldithiocarbamat,
(3) trans-S-(4-Fluorbenzyl)-N-4-[4-(dimethylaminomethyl)-phenyl]cyclohexyl-N-methyldithiocarbamat,
(4) trans-S-(4-Chlorbenzyl)-N-4-[4-(dimethylaminomethyl)-phenyl]cyclohexyl-N-methyldithiocarbamat,
(5) trans-O-(4-Chlorphenyl)-N-4-[4-(dimethylaminomethyl)-phenyl]cyclohexyl-N-methylcarbamat,
(6) cis-S-Benzyl-N-4-[4-(dimethylaminomethyl)phenyl]-cyclohexyl-N-methyldithiocarbamat,
(7) trans-O-(4-Chlorphenyl)-N-4-[4-(dimethylaminomethyl)-phenyl]cyclohexyl-N-methylthiocarbamat,
(8) trans-S-(4-Chlorphenyl)-N-4-[4-(dimethylaminomethyl)-phenyl]cyclohexyl-N-methylthiocarbamat,
(9) trans-O-(4-Chlorphenyl)-N-methyl-N-4-[4-fpiperidinomethyl)phenyl]cyclohexylcarbamat,
(10) trans-O-(4-Chlorphenyl)-N-methyl-N-4-[4-(pyrrolidinomethyl)phenyl]cyclohexylcarbamat,
(11) trans-O-(4-Chlorphenyl)-N-4-[4-(diethylaminomethyl)-phenyl]cyclohexyl-N-methylcarbamat,
(12) trans-O-(4-Chlorphenyl)-N-methyl-N-4-[4-(N-methyl-N-allylaminomethyl)phenyl]cyclohexylcarbamat,
(13) trans-O-(4-Chlorphenyl)-N-methyl-N-4-[4-(N-methyl-N-propargylaminomethyl)phenyl]cyclohexylcarbamat,
(14) trans-N-4-[4-(Dimethylaminomethyl)phenyl]cyclohexyl-N-methyl-O-(4-methylphenyl)carbamat,
(15) trans-N-4-[4-(Dimethylaminomethyl)phenyl]cyclohexyl-N-methyl-O-(4-methylphenyl)thiocarbamat,
(16) trans-N-Methyl-O-(4-methylphenyl)-N-4-[4-(piperidinomethyl)phenyl]cyclohexylthiocarbamat und
(17) trans-N-4-[4-(Dimethylaminomethyl)phenyl]cyclohexyl-O-(4-fluorphenyl)-N-methylcarbamat,
deren Gemische und deren Salze, insbesondere deren physiologisch verträglichen Säureadditionssalze, beispielsweise deren Hydrochloride, Methansulfonate oder Tartrate.

Die Verbindungen der allgemeinen Formel I lassen sich beispielsweise nach folgenden Methoden herstellen:
a) Umsetzung einer Verbindung der allgemeinen Formel 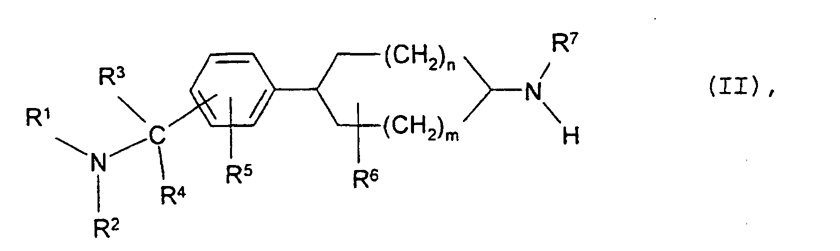 in der
   m ,n und R¹ bis R⁷ wie eingangs erwähnt definiert sind, mit einer Verbindung der allgemeinen Formel 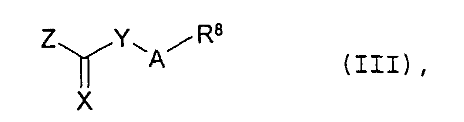 in der
   A , X, Y und R⁸ wie eingangs erwähnt definiert sind und Z eine Austrittsgruppe, beispielsweise ein Halogenatom, wie das Chlor-, Brom- oder Iodatom bedeutet,
   und, falls nötig, anschließende Abspaltung von Schutzgruppen.
   Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösemitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyl-diisopropylamin, N-Ethyl-dicyclohexylamin, 1,4-Diazabicyclo[2,2,2]octan oder 1,8-Diazabicyclo[5,4,0)undec-7-en, als Lösemittel beispielsweise Diethylether, Methylenchlorid, Dichlormethan, Ethylacetat, Toluol, Tetrahydrofuran, 1,4-Dioxan, Acetonitril, Dimethylformamid, Dimethylacetamid, N-Methyl-pyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.
   Stellt R² ein Wasserstoffatom dar, so wird die Reaktion zweckmäßigerweise so durchgeführt,daß zunächst eine Verbindung der allgemeinen Formel (II) ,in der R² eine Schutzgruppe, wie vorzugsweise den tert.Butyloxycarbonylrest, darstellt, zur Reaktion gebracht wird und nach beendeter Umsetzung die Schutzgruppe nach üblichen Methoden wieder abgespalten wird, beispielsweise durch Einwirkung von Trifluoressigsäure oder Chlorwasserstoff in Dioxan.
b) Zur Herstellung von Verbindungen der allgemeinen Formel (I), in der X und Y jeweils ein Schwefelatom bedeuten und m, n, A und R¹ bis R⁸ mit der Maßgabe wie eingangs erwähnt definiert sind, daß R⁸ keine gegebenenfalls substituierte Phenyl- oder Naphtylgruppe darstellt, falls A eine Einfachbindung bedeutet:
Umsetzung von Verbindungen der allgemeinen Formel (II), in der m, n und R¹ bis R⁷ wie eingangs erwähnt definiert sind, mit Schwefelkohlenstoff und anschließend mit einem Alkylierungsmittel der allgemeinen Formel 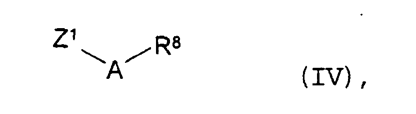 in der
   A und R⁸ mit der Maßgabe wie eingangs erwähnt definiert sind, daß R⁸ keine gegebenenfalls substituierte Phenyl- oder Naphtylgruppe darstellt, falls A eine Einfachbindung bedeutet, und
   Z¹ eine Austrittsgruppe, beispielsweise ein Halogenatom, wie das Chlor-, Brom- oder Iodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxyoder Naphthylsulfonyloxygruppe, wobei die Substituenten gleich oder verschieden sein können, bedeutet,
   und, falls nötig, anschließende Abspaltung von Schutzgruppen.

Stellt R² ein Wasserstoffatom dar, wird zweckmäßigerweise zunächst eine Verbindung der allgemeinen Formel (II), in der R² eine Schutzgruppe, beispielsweise einen tert. Butoxycarbonylrest darstellt, zur Reaktion gebracht und anschliessend die Schutzgruppe nach üblichen Methoden abgespalten, beispielsweise durch Trifluoressigsäure oder Chlorwasserstoff in Dioxan.

Stellt R² eine durch eine Hydroxygruppe substituierte Alkylgruppe dar, empfiehlt es sich, die Hydroxygruppe vor der Umsetzung zu schützen, beispielsweise durch den Tetrahydropyranylrest, der nach der Umsetzung wieder abgespalten wird, beispielsweise durch Trifluoressigsäure oder durch Chlorwasserstoff in Dioxan.

Die Umsetzung wird zweckmäßigerweise so durchgeführt, daß eine Verbindung der allgemeinen Formel (II) in einem geeigneten Lösungsmittel, beispielsweise in Tetrahydrofuran, zunächst in das Lithiumsalz überführt wird, beispielsweise mit n-Butyllithium bei einer Temperatur von -20 bis -10°C, und dann mit Schwefelkohlenstoff umgesetzt wird. Anschliessend wird eine Verbindung der allgemeinen Formel (IV) in einem geeigneten Lösungsmittel, beispielsweise in Tetrahydrofuran, Dimethylformamid oder einem Gemisch der beiden Lösungsmittel, zugegeben und die Umsetzung bei 20-60°C durchgeführt.

Bei den vorstehend beschriebenen Umsetzungen können gegebenenfalls vorhandene reaktive Gruppen wie Hydroxy-, Amino- oder Alkylamino- während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe und

als Schutzrest für eine Amino- oder Alkylaminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/-Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die nach den vorstehenden Verfahren hergestellten Verbindungen der allgemeinen Formel I lassen sich nach bekannten Methoden reinigen und isolieren, beispielsweise mittels Kristallisation, Destillation oder Chromatographie.

Ferner können die erhaltenen Verbindungen der allgemeinen Formel I gegebenenfalls in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971)) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder

Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

In den erfindungsgemäßen Verbindungen der allgemeinen Formel I können der an den Cycloalkylring gebundene Arylrest sowie das Stickstoffatom entweder äquatoriale oder axiale Anordnung einnehmen. Die Erfindung umfaßt sowohl die reinen Isomeren als auch die Gemische der verschiedenen Isomeren.

Die Ausgangsverbindungen der allgemeinen Formel II sind bekannt und lassen sich nach den in DE-A-4 438 020 und EP-A-0 599 203 beschriebenen Methoden herstellen.

Eine weitere Darstellungsmethode für Verbindungen der allgemeinen Formel II, in denen R³ bis R⁵ Wasserstoffatome bedeuten und der Phenylrest 1,4-disubstituiert ist, besteht darin, daß eine Verbindung der allgemeinen Formel V 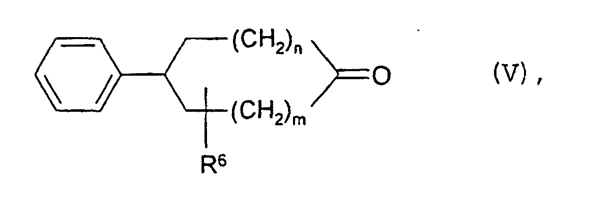 in der
m, n und R⁶ wie eingangs erwähnt definiert sind, nach den in DE-A-4 438 020 und EP-A-0 599 203 beschriebenen Methoden in eine Verbindung der Formel VI 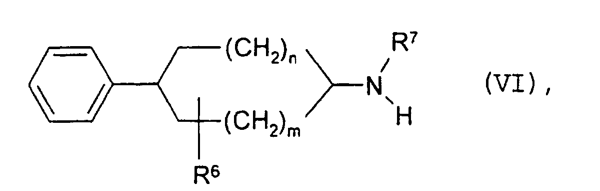 überführt wird, die anschließend, vorzugsweise nach Einführung einer Schutzgruppe am Stickstoffatom, beispielsweise einer Trifluoracetylgruppe oder 2.2.2-Trichlorethoxycarbonylgruppe, durch Chlormethylierung in eine Verbindung der Formel (VII) 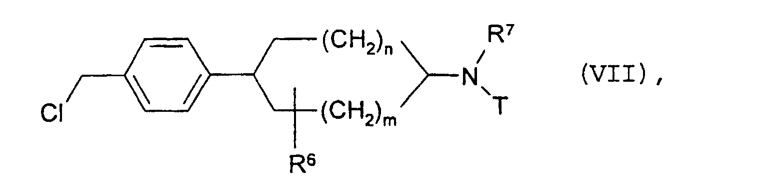 überführt wird, in der
m, n und R⁷ wie eingangs erwähnt definiert sind und T eine Schutzgruppe darstellt, beispielsweise die 2.2.2-Trichlorethoxycarbonyl- oder die Trifluoracetylgruppe, und abschliessend die Verbindung der Formel (VII) mit einem Amin der Formel H-NR¹R², in R¹ und R² wie eingangs erwähnt definiert sind, umgesetzt und nach Abspaltung der Schutzgruppe nach bekannten Methoden in eine Verbindung der Formel II überführt wird.

Die Verbindungen der allgemeinen Formel I besitzen interessante biologische Eigenschaften. Sie stellen Inhibitoren der Cholesterolbiosynthese, insbesondere Inhibitoren des Enzyms 2,3-Epoxisqualen-Lanosterol-Cyclase dar. Aufgrund ihrer biologischen Eigenschaften sind sie geeignet zur Behandlung von Krankheiten, bei denen die Cholesterol-Biosynthese eine Rolle spielt, insbesondere zur Behandlung und Prophylaxe der Hypercholesterolämie, der Hyperlipoproteinämie und der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens, Gangrän und andere.

Zur Behandlung dieser Erkrankungen können die Verbindungen der allgemeinen Formel I dabei entweder alleine zur Monotherapie eingesetzt werden oder in Kombination mit anderen cholesteroloder lipidsenkenden Substanzen zur Anwendung gelangen, wobei die Verbindungen vorzugsweise als orale Formulierung, gegebenenfalls auch in Form von Suppositorien als rektale Formulierung verabreicht werden können. Als Kombinationspartner kommen dabei beispielsweise in Frage:
- gallensäurebindende Harze wie z. B. Cholestyramin, Cholestipol und andere,
- Verbindungen, die die Cholesterolresorption hemmen, wie z. B. Sitosterol und Neomycin,
- Verbindungen, die über einen anderen Mechanismus als Hemmung der 2,3-Epoxisqualen-Lanosterol-Cyclase in die Cholesterolbiosynthese eingreifen, wie z. B. HMG-CoA-Reduktaseinhibitoren wie Lovastatin, Simvastatin, Pravastatin, Fluvastatin, Atorvastatin, Cerivastatin und andere,
- Squalen-Epoxidaseinhibitoren wie beispielsweise NB 598 und analoge Verbindungen sowie
- Squalen-Synthetaseinhibitoren wie beispielsweise Vertreter der Klasse der Isoprenoid-(phosphinylmethyl)phosphonate und Squalestatin.

Als weitere mögliche Kombinationspartner sind noch zu erwähnen die Klasse der Fibrate, wie Clofibrat, Bezafibrat, Gemfibrozil und andere, Nikotinsäure, ihre Derivate und Analoge wie beispielsweise Acipimox, sowie Probucol.

Desweiteren sind die Verbindungen der allgemeinen Formel I geeignet zur Behandlung von Erkrankungen, die mit überhöhter Zellproliferation im Zusammenhang stehen. Cholesterol ist ein essentieller Zellbestandteil und muß für die Zellproliferation, d. h. Zellteilung, in ausreichender Menge vorhanden sein. Die Inhibierung der Zellproliferation durch Inhibierung der Cholesterolbiosynthese ist am Beispiel der glatten Muskelzellen mit dem HMG-CoA-Reduktaseinhibitor des Mevinolintyps Lovastatin, wie eingangs erwähnt, beschrieben.

Als Beispiele für Erkrankungen, die mit überhöhter Zellproliferation zusammenhängen sind zunächst Tumorerkrankungen zu nennen. In Zellkultur- und in-vivo-Experimenten wurde gezeigt, daß die Senkung des Serumcholesterols oder der Eingriff in die Cholesterolbiosynthese durch HMG-CoA-Reduktaseinhibitoren das Tumorwachstum vermindert (Lancet 339, 1154-1156 [1992]). Die erfindungsgemäßen Verbindungen der Formel I sind deshalb aufgrund ihrer cholesterolbiosyntheseinhibitorischen Wirkung potentiell für die Behandlung von Tumorerkrankungen geeignet. Sie können dabei alleine oder zur Unterstützung bekannter Therapieprinzipien Verwendung finden.

Als weitere Beispiele sind hyperproliferative Hauterkrankungen wie beispielsweise Psoriasis, Basalzellkarzinome, Plattenepithelkarzinome, Keratosis und Keratinisierungsstörungen zu nennen. Der hier verwendete Ausdruck "Psoriasis" bezeichnet eine hyperproliferativ entzündliche Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, verdickte Epidermis, abnormale Keratinisierung entzündlicher Zellinfiltrate in die Dermisschicht und polymorphonucleäre Leukozyteninfiltration in die Epidermis, die eine Zunahme des Basalzellzyklus bedingt, gekennzeichnet. Zusätzlich sind hyperkeratotische und parakeratotische Zellen anwesend. Der Ausdruck "Keratosis", "Basalzellkarzinome", "Plattenepithelkarzinome" und "Keratinisierungsstörungen" bezieht sich auf hyperproliferative Hauterkrankungen, bei denen der Regulierungsmechanismus für die Proliferation und Differenzierung der Hautzellen unterbrochen ist.

Die Verbindungen der Formel I sind wirksam als Antagonisten der Hauthyperproliferation, d. h. als Mittel, die die Hyperproliferation menschlicher Keratinozyten hemmen. Die Verbindungen sind infolgedessen als Mittel zur Behandlung hyperproliferativer Hauterkrankungen wie Psoriasis, Basalzellkarzinomen, Keratinisierungsstörungen und Keratosis geeignet. Zur Behandlung dieser Krankheiten können die Verbindungen der Formel I entweder oral oder topisch appliziert werden, wobei sie entweder alleine in Form der Monotherapie oder in Kombination mit bekannten Wirkstoffen eingesetzt werden können.

Des weiteren zu nennen sind durch chirurgische Maßnahmen wie PTCA (perkutane transluminale coronare Angioplastie) oder Bypass-Operationen ausgelöste hyperproliferative Gefäßerkrankungen wie Stenosen und Gefäßverschlüsse, die auf der Proliferation glatter Muskelzellen beruhen. Wie eingangs erwähnt läßt sich diese Zellproliferation bekanntlich durch HMG-CoA-Reduktaseinhibitoren vom Mevinolintyp, wie Lovastatin, unterdrücken. Aufgrund ihrer inhibitorischen Wirkung auf die Cholesterolbiosynthese sind auch die Verbindungen der allgemeinen Formel I geeignet zur Behandlung und Prophylaxe dieser Erkrankungen, wobei sie entweder alleine oder in Kombination mit bekannten Wirkstoffen, wie z. B. intravenös appliziertes Heparin, vorzugsweise in oraler Applikation Verwendung finden können.

Eine weitere Einsatzmöglichkeit der erfindungsgemäßen Verbindungen der allgemeinen Formel I ist die Prophylaxe und Behandlung von Gallensteinleiden. Die Gallensteinbildung wird dadurch ausgelöst, daß die Cholesterolkonzentration in der Galle die maximale Löslichkeit des Cholesterols in der Gallenflüssigkeit überschreitet, wodurch es zur Ausfällung des Cholesterols in Form von Gallensteinen kommt. Lipidsenker aus der Klasse der Fibrate führen zu einer erhöhten Ausscheidung von Neutralsteroiden über die Galle und erhöhen die Neigung zur Gallensteinbildung.

Im Gegensatz dazu führen Cholesterolbiosynthesehemmer wie Lovastatin oder Pravastatin zu keiner erhöhten Gallensteinbildung, sondern können im Gegenteil eine Reduktion der Cholesterolkonzentration in der Galle bewirken und damit den sogenannten lithogenen Index, ein Maß für die Wahrscheinlichkeit der Gallensteinbildung, vermindern. Dies ist beschrieben in Gut 31, 348-350 [1990] sowie in Z. Gastroenterol. 29, 242-245 [1991].

Darüber hinaus ist in Gastroenterology 102, No. 4, Pt. 2, A 319 [1992] die Wirksamkeit von Lovastatin bei der Auflösung von Gallensteinen, insbesondere in Kombination mit Ursodeoxycholsäure beschrieben. Aufgrund ihrer Wirkungsweise sind die Verbindungen der allgemeinen Formel I deshalb auch für die Prophylaxe und Behandlung von Gallensteinleiden von Bedeutung. Sie können dabei entweder allein oder in Kombination mit bekannten Therapien wie beispielsweise der Behandlung mit Ursodeoxycholsäure oder der Schockwellenlithotripsie vorzugsweise in oraler Applikation Verwendung finden.

Schließlich sind die Verbindungen der allgemeinen Formel I geeignet zur Therapie von Infektionen durch pathogene Pilze wie z. B. Candida albicans, Aspergillus niger, Trichophyton mentagrophytes, Penicillium sp., Cladosporium sp. und andere. Wie bereits eingangs erwähnt ist das Endprodukt der Sterolbiosynthese im Pilzorganismus nicht Cholesterol, sondern das für die Integrität und Funktion der Pilzzellmembranen essentielle Ergosterol. Die Inhibierung der Ergosterolbiosynthese führt deshalb zu Wachstumsstörungen und gegebenenfalls zur Abtötung der Pilzorganismen. Zur Behandlung von Mykosen können die Verbindungen der allgemeinen Formel I entweder oral oder topisch appliziert werden. Dabei können sie entweder alleine oder in Kombination mit bekannten antimykotischen Wirkstoffen eingesetzt werden, insbesondere mit solchen, die in andere Stufen der Sterolbiosynthese eingreifen, wie beispielsweise den Squalen-Epoxidasehemmern Terbinafin und Naftifin oder den Lanosterol-14a-Demethylaseinhibitoren vom Azol-Typ wie beispielsweise Ketoconazol und Fluconazol.

Eine weitere Verwendungsmöglichkeit der Verbindungen der allgemeinen Formel I betrifft die Anwendung in der Geflügelhaltung. Die Senkung des Cholesterolgehaltes von Eiern durch Verabreichung des HMG-CoA-Reduktaseinhibitors Lovastatin an Legehennen ist beschrieben (FASEB Journal 4, A 533, Abstracts 1543 [1990]). Die Erzeugung cholesterolarmer Eier ist von Interesse, da die Cholesterolbelastung des Körpers durch Eier mit reduziertem Cholesterolgehalt ohne eine Änderung der Ernährungsgewohnheiten vermindert werden kann. Aufgrund ihrer inhibitorischen Wirkung auf die Cholesterolbiosynthese können die Verbindungen der allgemeinen Formel I auch in der Geflügelzucht zur Erzeugung cholesterolarmer Eier Verwendung finden, wobei die Substanzen vorzugsweise als Zusatz zum Futter verabreicht werden.

Die biologische Wirkung von Verbindungen der allgemeinen Formel I wurde nach folgenden Methoden bestimmt:

### I. Messung der Hemmung des ¹⁴C-Acetat-Einbaus in die mit Digitonin fällbaren Steroide:

Die Untersuchung der Hemmwirkung wurde nach der in J. Lipid. Res. 37, 148-157 [1996] beschriebenen Methode bei Testkonzentrationen von 10⁻⁸ und 10⁻⁹ Mol/l durchgeführt.

Beispielhaft werden die Testergebnisse der folgenden Verbindungen (A) bis (R) der allgemeinen Formel I sowie der Vergleichssubstanzen (U), (V) und (W) bei diesen Testkonzentrationen angegeben:
(A) trans-S-(4-Chlorphenyl)-N-4-[4-(dimethylaminomethyl)-phenyl]cyclohexyl-N-methyldithiocarbamat,
(B) trans-S-(3-Fluorbenzyl)-N-4-[4-(dimethylaminomethyl)-phenyl]cyclohexyl-N-methyldithiocarbamat-hydrochlorid,
(C) trans-S-(4-Fluorbenzyl)-N-4-[4-(dimethylaminomethyl)-phenyl]cyclohexyl-N-methyldithiocarbamat-hydrochlorid,
(D) trans-S-(4-Chlorbenzyl)-N-4-[4-(dimethylaminomethyl)-phenyl]cyclohexyl-N-methyldithiocarbamat-hydrochlorid,
(E) trans-O-(4-Chlorphenyl)-N-4-[4-(dimethylaminomethyl)-phenyl]cyclohexyl-N-methylcarbamat-hydrochlorid,
(F) cis-S-Benzyl-N-4-[4-(dimethylaminomethyl)phenyl]-cyclohexyl-N-methyldithiocarbamat-hydrochlorid,
(G) trans-O-(4-Chlorphenyl)-N-4-[4-(dimethylaminomethyl)-phenyl]cyclohexyl-N-methylthiocarbamat-hydrochlorid,
(H) trans-S-(4-Chlorphenyl)-N-4-[4-(dimethylaminomethyl)-phenyl]cyclohexyl-N-methylthiocarbamat-hydrochlorid,
(I) trans-O-(4-Chlorphenyl)-N-methyl-N-4-[4-(piperidinomethyl)phenyl]cyclohexylcarbamat-hydrochlorid,
(K) trans-O-(4-Chlorphenyl)-N-methyl-N-4-[4-(pyrrolidinomethyl)phenyl]cyclohexylcarbamat-hydrochlorid,
(L) trans-O-(4-Chlorphenyl)-N-4-[4-(diethylaminomethyl)-phenyl]cyclohexyl-N-methylcarbamat-hydrochlorid,
(M) trans-O-(4-Chlorphenyl)-N-methyl-N-4-[4-(N-methyl-N-allylaminomethyl)phenyl]cyclohexylcarbamat,
(N) trans-O-(4-Chlorphenyl)-N-methyl-N-4-[4-(N-methyl-N-propargylaminomethyl)phenyl]cyclohexylcarbamat,
(O) trans-N-4-(4-(Dimethylaminomethyl)phenyl]cyclohexyl-N-methyl-O-(4-methylphenyl)carbamat-hydrochlorid,
(P) trans-N-4-[4-(Dimethylaminomethyl)phenyl]cyclohexyl-N-methyl-O-(4-methylphenyl)thiocarbamat-hydrochlorid,
(Q) trans-N-Methyl-O-(4-methylphenyl)-N-4-[4-(piperidinomethyl)phenyl]cyclohexylthiocarbamat-hydrochlorid,
(R) trans-N-4-[4-(Dimethylaminomethyl)phenyl]cyclohexyl-O-(4-fluorphenyl)-N-methylcarbamat-hydrochlorid,
(U) 1-(4-Chlorbenzoyl)-4-[4-(2-oxazolin-2-yl)-benzyl-iden] -piperidin (EP-A-0 596 326, S. 16, dort Verbindung A; J. Lipid. Res. 38, 564-575 [1997]),
(V) trans-N-(4-Chlorbenzoyl)-N-methyl-[4-(4-dimethylamino)-methyl)phenyl]cyclohexylamin (DE-A-44 38 020; J. Lipid. Res. 37, 148-157 [1996]) und
(W) trans-O-(p-Tolylacetyl)-4-(4-dimethylaminomethylphenyl)-cyclohexanol (WO 95/29148, S. 28, dort Verbindung I).

Die Prozentwerte, um die obigen Verbindungen den ¹⁴C-Acetat-Einbau hemmen, sind in Tabelle 1 angegeben.

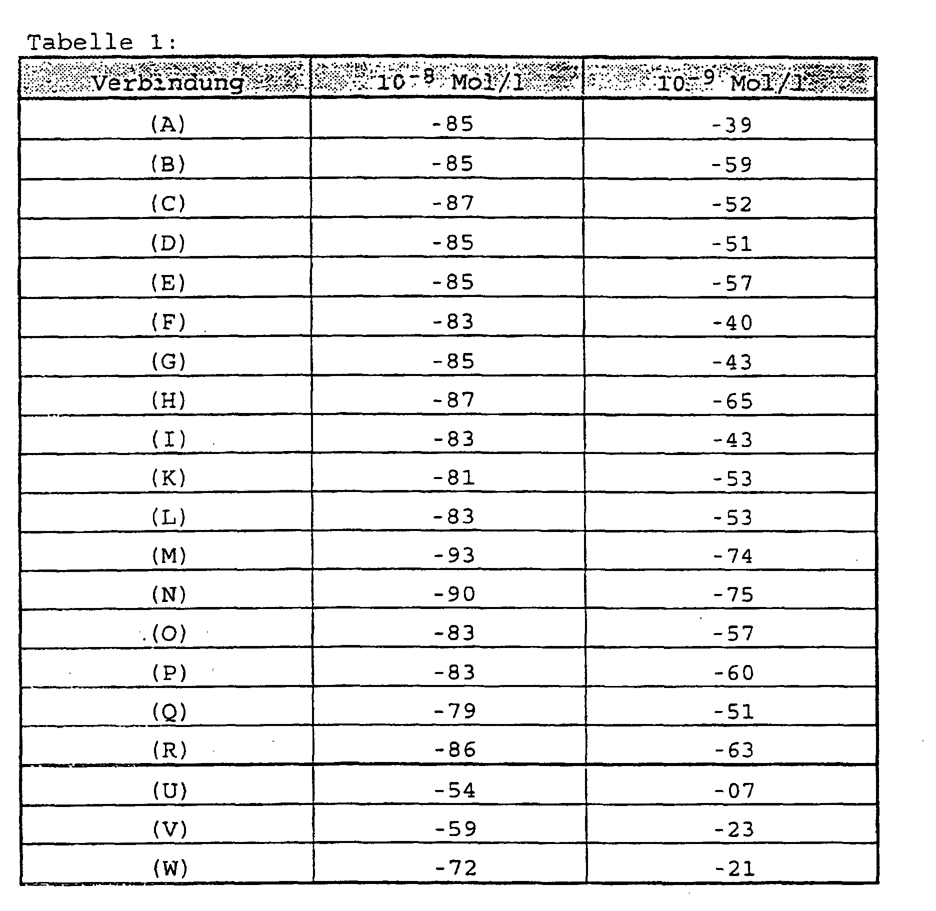

Von den Verbindungen E, G, H, O und R wurden die IC₅₀-Werte bestimmt. Diese sind zusammen mit den IC₅₀-Werten der Verbindungen U, V und W in Tabelle 2 angegeben.

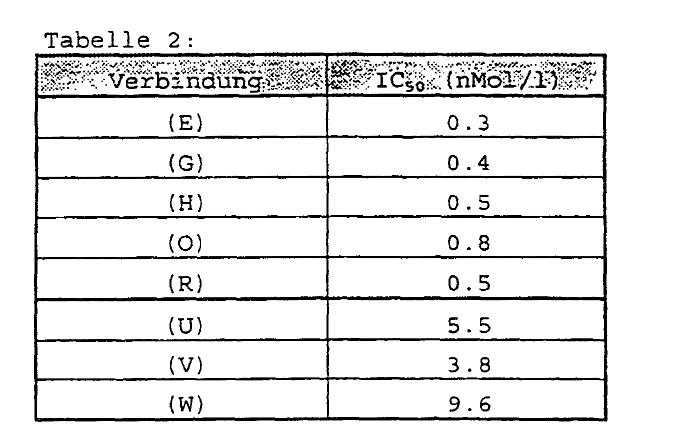

Aus Tabelle 2 ist eine signifikante Überlegenheit der erfindungsgemäßen Verbindungen gegenüber den vorbeschriebenen Vergleichssubstanzen ersichtlich.

### II. Messung der in-vivo Wirkung an der Ratte nach oraler Gabe

Die Inhibierung des Enzyms 2,3-Epoxisqualen-Lanosterol-Cyclase bewirkt eine Erhöhung der 2,3-Epoxisqualenspiegel in Leber und Plasma. Die Menge an gebildetem 2,3-Epoxisqualen dient daher als direktes Maß für die Wirkstärke am Ganztier.Die Bestimmung wurde nach der in J. Lipid. Res. 38, 564-575, [1997] beschriebenen Methode nach t = 3 bzw. 8 Stunden nach Substanzgabe mit Konzentrationen von c = 0.01, 0.03, 0.1, 0.3 und 1.0 mg/kg durchgeführt. In der folgenden Tabelle 3 sind beispielhaft die Ergebnisse für die vorstehend erwähnten Substanzen A, B, C, E, G, H, O und R angegeben.

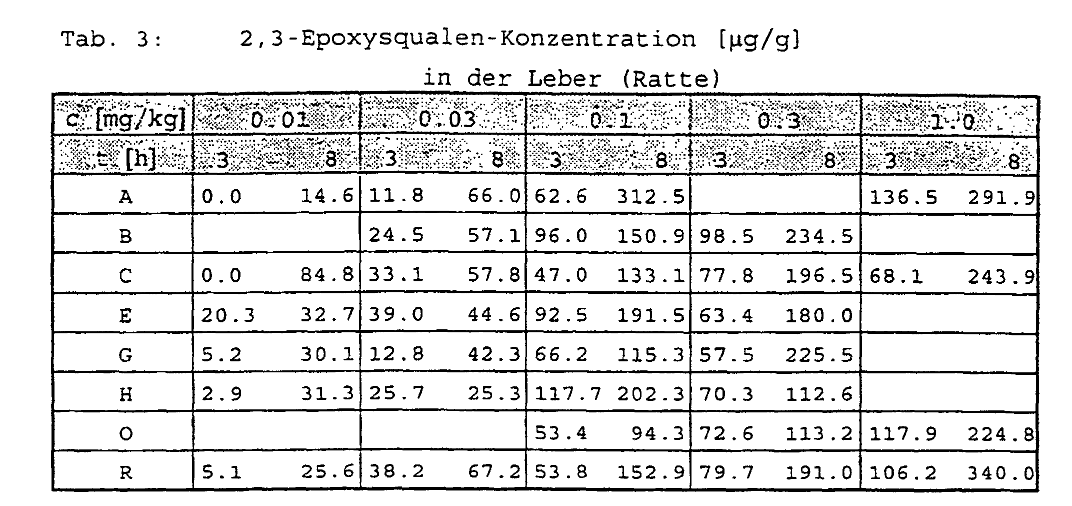

Bei den Kontrolltieren traten unter diesen Bedingungen keine messbaren 2,3-Epoxisqualenspiegel auf.

### III. Lipidsenkung am normolipämischen Goldhamster

Diese Bestimmung wurde nach der in J. Lipid. Res. 38, 564-575, (1997) beschriebenen Methode durchgeführt. Am Ende des Versuchs wurde Gesamtcholesterol, β-Lipoprotein-Cholesterol sowie HDL-Cholesterol bestimmt und mit den Werten einer ohne Testsubstanz gefütterten Kontrollgruppe verglichen.

Geprüft wurde die lipidsenkende Wirkung der vorstehend erwähnten Verbindungen E, G und I.

Die Ergebnisse sind in Tabelle 4 zusammengefaßt.

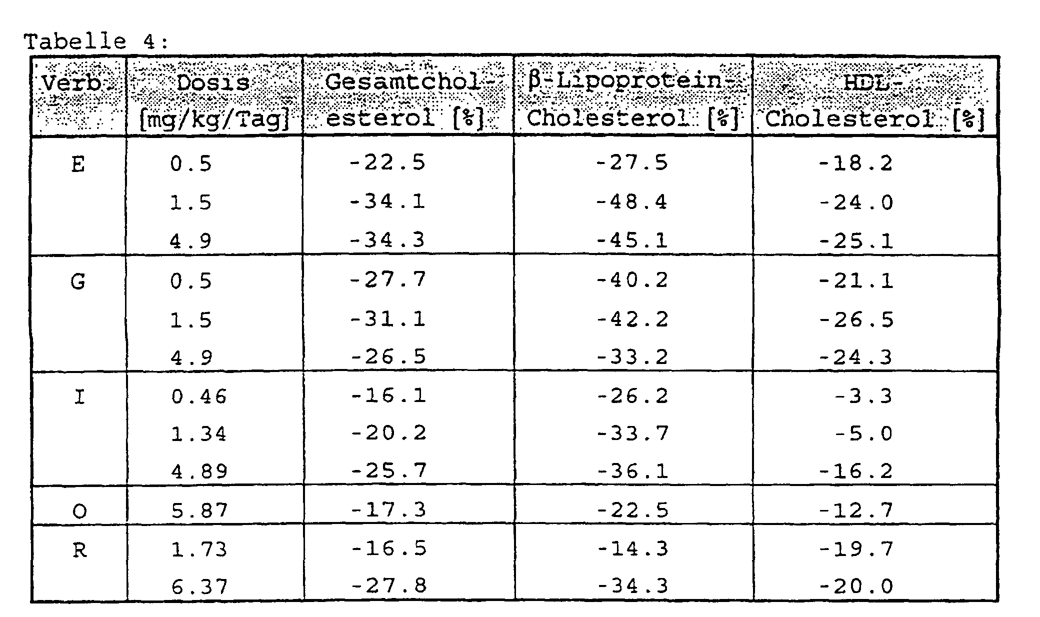

Unter diesen Bedingungen zeigten die Verbindungen keine toxischen Effekte.

### IV. Hemmung der Zellproliferation

HaCat-Zellen (humane Keratinozyten) werden mit einer Dichte von 10 000 Zellen pro well in eine 96 well Mikrotiterplatte ausgesät. Als Kulturmedium dient Dulbeccos modified eagle medium (DMEM) unter Zusatz von 10 % Kälberserum. Die Zellen werden zwei Tage im Brutschrank inkubiert, anschließend wird die in Dimethylsulfoxyd gelöste und mit Kulturmedium verdünnte Testsubstanz zugegeben. Nach zwei weiteren Tagen Inkubation im Brutschrank wird 5-Brom-2'-deoxyuridin zugegeben und nach Vorschrift (Boehringer Mannheim, Cell Proliferation ELISA, BrdU(colorimetric)) das Testergebnis bestimmt. Die BrdU-Messung erfolgt bei 380-490 nm in Plate Reader der Firma Bio Rad.

Geprüft wurde die proliferationshemmende Wirkung der Verbindungen E, G und H. Es wurde der Mittelwert von jeweils drei Messungen errechnet und das Ergebnis in % der Kontrolle angegeben (Tabelle 5).

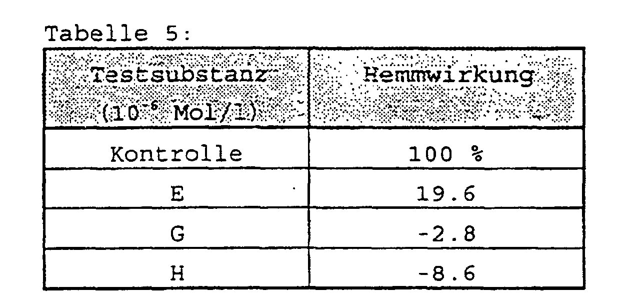

### V. Bestimmung der fungistatischen Wirkung

Die fungistatische Wirkung wurde über den Reihenverdünnungstest (Mikrotitersystem) bestimmt. Als Nährmedium diente Sabouraud-Bouillon. Das Inoculum betrug ca. 10⁴ bis 10⁵ KBE/ml (KBE = koloniebildende Einheiten); die Bebrütungszeit betrug 2 bis 4 Tage bei 26°C.

Es wurde die niedrigste Konzentration, die sichtbares Wachstum nicht mehr zuließ (minimale Hemmkonzentration MHK), bestimmt.

Geprüft wurden die vorstehend erwähnten Verbindungen B, E, G, I, K, L, N, Q und R. Die Ergebnisse sind in der folgenden Tabelle 6 zusammengefaßt. Die MHK ist in µg/ml angegeben.

Folgende Testkeime wurden verwendet:

| Testkeim | Abkürzung |
|---|---|
| Cand. albicans ATCC 10231 | Cand. |
| Sacch. carlsbergensis ATCC 9080 | Sacc. |
| Rhod. rubra 49 | Rhod. |
| Asp. niger ATCC 16404 | Asp. |
| Trich. mentagrophytes ATCC 9129 | Trich. |
| Pen.notatum CBS 19746 | Pen. |

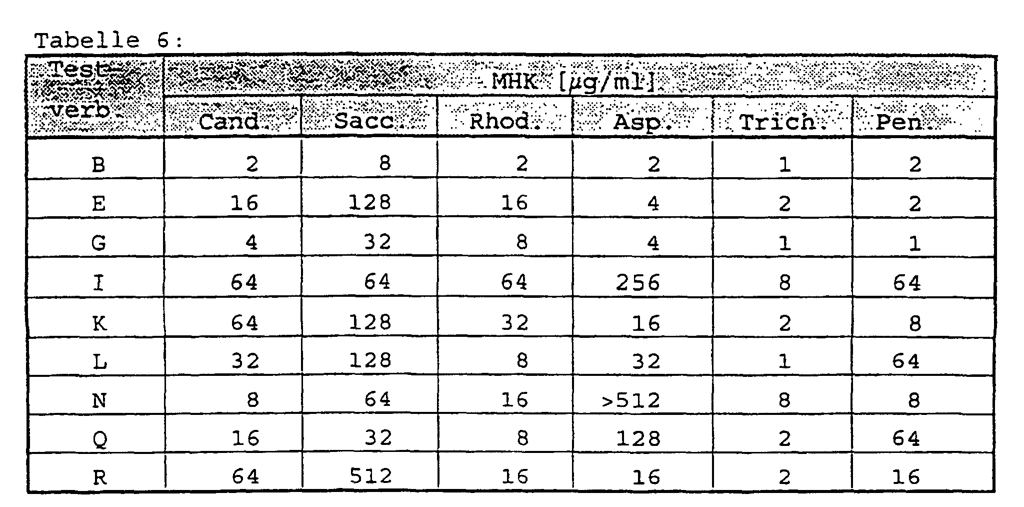

Zur pharmazeutischen Anwendung lassen sich die Verbindungen der allgemeinen Formel I in an sich bekannter Weise in die üblichen pharmazeutischen Zubereitungsformen für die orale, rektale und topische Verabreichung einarbeiten.

Formulierungen für die orale Verabreichung umfassen beispielsweise Tabletten, Dragées und Kapseln. Für die rektale Verabreichung kommen vorzugsweise Suppositorien in Betracht. Die Tagesdosis beträgt zwischen 0.1 und 200 mg für einen Menschen mit 60 kg Körpergewicht, bevorzugt ist jedoch eine Tagesdosis von 1 bis 100 mg für einen Menschen mit 60 kg Körpergewicht. Die Tagesdosis wird vorzugsweise in 1 bis 3 Einzelgaben aufgeteilt.

Bei topischer Anwendung können die Verbindungen in Zubereitungen, die etwa 1 bis 1000 mg, insbesondere 10 bis 300 mg Wirkstoff pro Tag enthalten, verabreicht werden. Die Tagesdosis wird vorzugsweise in 1 bis 3 Einzelgaben aufgeteilt.

Topische Formulierungen umfassen Gele, Cremes, Lotionen, Salben, Puder, Aerosole und andere herkömmliche Formulierungen zur Anwendung von Heilmitteln auf der Haut. Die Wirkstoffmenge für die topische Anwendung beträgt 1 bis 50 mg pro Gramm Formulierung, vorzugsweise jedoch 5 bis 20 mg pro Gramm Formulierung. Neben der Anwendung auf der Haut können die topischen Formulierungen der vorliegenden Erfindung auch angewandt werden bei der Behandlung von Schleimhäuten, die der topischen Behandlung zugänglich sind. Beispielsweise können die topischen Formulierungen auf die Schleimhäute des Mundes, des unteren Colons und andere aufgebracht werden.

Zur Anwendung in der Geflügelzucht zur Erzeugung cholesterolarmer Eier werden die Wirkstoffe der allgemeinen Formel I den Tieren nach den üblichen Methoden als Zusatz zu geeigneten Futtermitteln verabreicht. Die Konzentration der Wirkstoffe im Fertigfutter beträgt normalerweise 0.01 bis 1%, vorzugsweise jedoch 0.05 bis 0.5%.

Die Wirkstoffe können als solche dem Futter zugesetzt werden. So enthalten die erfindungsgemäßen Futtermittel für Legehennen neben dem Wirkstoff und gegebenenfalls neben einer üblichen Vitamin-Mineral-Mischung beispielsweise Mais, Sojabohnenmehl, Fleischmehl, Futterfett und Sojaöl. Zu diesem Futter wird eine der eingangs erwähnten Verbindungen der Formel I als Wirkstoff in einer Konzentration von 0.01 bis 1%, vorzugsweise jedoch 0.05 bis 0.5% zugemischt.

Die nachfolgenden Beispiele dienen der näheren Erläuterung der Erfindung. Die angegebenen R_{f}-Werte wurden an Fertigplatten der Firma E.Merck, Darmstadt bestimmt und zwar an:
a) Aluminiumoxid F-254 (Typ E)
b) Kieselgel 60 F-254

### Beispiele zur Herstellung der Ausgangsmaterialien:

### Beispiel A

### trans-N-Methyl-4-[4-(piperidinomethyl)phenyl]cyclohexylamin

26 g (0.15 Mol) 4-Phenylcyclohexanon werden mit 160 ml einer 6 %-igen Methylaminlösung in Toluol in Gegenwart von 30 g Molekularsieb M 3 Å über Nacht im verschlossenen Kolben gerührt. Nach Filtrieren und Einengen wird der Rückstand in 200 ml Methanol gelöst und bei -20°C portionsweise mit 7.8 g Natriumborhydrid versetzt. Nach beendeter Zugabe wird bei Raumtemperatur über Nacht gerührt. Nach Verdampfen des Methanols wird in 150 ml Eiswasser aufgenommen und mit konz. Salzsäure stark angesäuert. Nach Sättigen mit Kochsalz wird das ausgefallene Hydrochlorid abgesaugt, die Mutterlauge mit 6N-Natronlauge stark alkalisch gestellt, mit Essigsäureethylester extrahiert, getrocknet und eingedampft. Das abgesaugte Hydrochlorid wird in einem Wasser-Essigsäureethylester-Gemisch suspendiert und unter Kühlung mit 6N-Natronlauge stark alkalisch gestellt. Die Essigsäureethylesterphase wird mit Wasser und gesättigter Kochsalzlösung gewaschen, getrocknet und eingedampft. Kristallisation des Rückstands aus Petrolether ergibt 15.1 g trans-N-Methyl-4-phenylcyclohexylamin (farblose Kristalle). Die Petrolethermutterlauge wird eingedampft und mit dem Eindampfrückstand der Hydrochloridfällung vereinigt. Trennung an Aluminiumoxid (Aktivitätsstufe III, Essigsäureethylester/Petrolether = 1:1 bis 2:1, v:v) ergibt 2.4 g cis-N-Methyl-4-phenylcyclohexylamin, 4.4 g trans-Verbindung sowie 5.1 g eines Gemisches aus cis- und trans-N-Methyl-4-phenylcyclohexylamin. Gesamtausbeute an trans-N-Methyl-4-phenylcyclohexylamin: 19.5 g (68.6 % d.Th.), farblose Kristalle.

19.5 g (0.103 mol) trans-N-Methyl-4-phenylcyclohexylamin und 12.1 g (0.12 mol) Triethylamin werden in 200 ml Essigsäureethylester gelöst. Unter Eiskühlung werden 24.0 g (0.113 mol) Chlorameisensäure-2.2.2-trichlorethylester in 50 ml Essigsäureethylester zugetropft und 2 Stunden nachgerührt. Nach Stehen über Nacht bei Raumtemperatur wird mit Essigsäureethylester verdünnt und mit Wasser, verdünnter Salzsäure, nochmals mit Wasser und abschliessend mit gesättigter Kochsalzlösung gewaschen. Der nach Trocknen (Magnesiumsulfat) und Eindampfen erhaltene Rückstand wird in der Hitze in einem Gemisch aus Petrolether und Essigsäureethylester (10:1, v:v) gelöst. Langsames Abkühlen, zuletzt bei 0°C, ergibt 34.3 g (91.3% d.Th.) trans-N-Methyl-N-2.2.2-trichlor-ethoxycarbonyl-4-phenylcyclohexylamin als farblose Kristalle vom Schmelzpunkt 84-86 C.

Weitere 1.1 g (2.9 % d.Th.) dieses Produkts werden nach Eindampfen der Mutterlauge und Reinigung des Rückstands an Kieselgel (Petrolether/Essigsäureethylester =10:1 bis 5:1, v:v) erhalten.

35.4 g (0.097 mol) dieser Verbindung werden in 950 ml Dichlormethan gelöst. Nach Zugabe von 30.7 g (0.345 mol) Paraformaldehyd und 30.7 g (0.226 mol) Zinkchlorid wird Chlorwasserstoff eingeleitet (2 Stunden bei Raumtemperatur) und über Nacht bei Raumtemperatur gerührt. Überschüssiger Chlorwasserstoff wird im Stickstoffstrom entfernt und das Reaktionsgemisch in gekühlte, wässrige Dinatriumhydrogenphosphatlösung gegossen. Die organische Phase wird abgetrennt, die wässrige Phase zweimal mit Methylenchlorid extrahiert, die organischen Phasen vereinigt, mit Wasser gewaschen, mit Magnesiumsulfat getrocknet und eingedampft. Der Rückstand wird durch Chromatographie an Kieselgel (Petrolether/Essigsäureethylester = 20:1 bis 15:1, v:v) gereinigt. Neben 14.0 g (39.5 % d. Th.) unverändertem Ausgangsmaterial erhält man 19.0 g (47.4 % d. Th.) trans-4-(4-Chlormethylphenyl)-N-methyl-N-2.2.2-trichlorethoxycarbonyl-cyclohexylamin als farblose Kristalle vom R_{f}-Wert 0.38 (Kieselgel, Petrolether/Essigsäureethylester 10:1, v:v).

830 mg (2mmol) dieser Verbindung werden in einem Gemisch aus jeweils 10ml Tetrahydrofuran und Ethanol gelöst, 510 mg (6mmol) Piperidin zugegeben, über Nacht bei Raumtemperatur und anschliessend 3 Stunden bei 50°C gerührt. Nach Abkühlen wird auf Wasser gegossen und dreimal mit Essigsäureethylester extrahiert. Die organische Phase wird mit Wasser und gesättigter Kochsalzlösung gewaschen, getrocknet (Magnesiumsulfat) und eingedampft. Reinigung des Rückstands durch Chromatographie (Aluminiumoxid, Aktivitätsstufe III, Petrolether/Essigsäureethylester = 10:1) ergibt 750 mg (81.2 % d.Th.) trans-N-Methyl-N-2.2.2-trichlorethoxycarbonyl-4-[4-(piperidinomethyl)-phenyl]cyclohexylamin (schwach gelbliche Kristalle), R_{f}-Wert 0.52 (Aluminiumoxid, Petrolether/Essigsäureethylester 5:1).

730 mg (1.58 mmol) dieser Verbindung werden in 1 ml Eisessig und 5 ml Wasser gelöst Bei Raumtemperatur werden 1.5 g Zinkpulver in einer Portion zugegeben (nach kurzer Zeit starkes Schäumen). Es wird 30 Minuten bei Raumtemperatur, dann 1 Stunde bei 50°C nachgerührt, abgekühlt, Wasser und Essigsäureethylester zugegeben und mit 6N-Natronlauge stark alkalisch gestellt. Nach Filtration wird die organische Phase abgetrennt, mit Wasser und gesättigter Kochsalzlösung gewaschen, getrocknet (Magnesiumsulfat) und eingedampft. Man erhält 340 mg (75.1 % d Th.) trans-N-Methyl-4-[4-(piperidinomethyl)-phenyl]cyclohexylamin (farblose Kristalle), R_{f}-Wert 0.3 (Aluminiumoxid, Methylenchlorid/Methanol 40:1, v:v).

### Analog werden erhalten:

(1) trans-N-Methyl-4-[4-(pyrrolidinomethyl)phenyl]-cyclohexylamin,
   aus 4-Phenylcyclohexanon und Pyrrolidin; farblose Kristalle; R_{f}-Wert 0.44 (Aluminiumoxid, Petrolether/Essigsäureethylester 5:1, v:v)
(2) trans-4-[4-(Diethylaminomethyl)phenyl]-N-methylcyclohexylamin,
   aus 4-Phenylcyclohexanon und Diethylamin; farbloser Feststoff; R_{f}-Wert 0.25 (Aluminiumoxid, Methylenchlorid/Methanol 40:1, v:v)
(3) trans-4-[4-[(2-Hydroxyethyl)methylaminomethyl]phenyl]-N-methylcyclohexylamin,
   aus 4-Phenylcyclohexanon und N-Methylethanolamin; farbloser Feststoff; R_{f}-Wert 0.33 (Aluminiumoxid, Methylenchlorid/Methanol 20:1, v:v)
(4) trans-4-[4-(Di-n-hexylaminomethyl)phenyl]-N-methylcyclohexylamin,
   aus 4-Phenylcyclohexanon und Di-n-hexylamin; farbloser Feststoff; R_{f}-Wert 0.26 (Aluminiumoxid, Methylenchlorid/Methanol 40:1, v:v)
(5) trans-4-[4-(Di-sec-butylaminomethyl)phenyl]-M-methylcyclohexylamin,
   aus 4-Phenylcyclohexanon und Di-sec-butylamin; farbloser Feststoff; R_{f}-Wert 0.58 (Aluminiumoxid, Methylenchlorid/Methanol 20:1, v:v)
(6) trans-N-Methyl-4-[4-(N-methylallylaminomethyl)phenyl]-cyclohexylamin,
   aus 4-Phenylcyclohexanon und N-Methylallylamin;, farbloser Feststoff; R_{f}-Wert 0.22 (Aluminiumoxid, Methylenchlorid/Methanol 40:1, v:v)
(7) trans-N-Methyl-4-[4-(N-methylpropargylaminomethyl)phenyl]-cyclohexylamin,
   aus 4-Phenylcyclohexanon und N-Methylpropargylamin; farblose Kristalle; R_{f}-Wert 0.23 (Aluminiumoxid, Methylenchlorid/Methanol 40:1, v:v
(8) cis/trans-N-Methyl-4-[4-(morpholinomethyl)phenyl]cyclohexylamin,
   aus 4-Phenylcyclohexanon und Morpholin; farbloser Feststoff; R_{f}-Werte 0.26 und 0.48 (Aluminiumoxid, Petrolether/Essigsäureethylester = 5:1, v:v)

### Beispiele zur Herstellung der Endprodukte:

### Beispiel 1

### trans-O-(4-Chlorphenyl)-N-4-[4-(dimethylaminomethyl)phenyl]-cyclohexyl-N-methylcarbamat-hydrochlorid

250 mg (1 mmol) trans-4-[4-(Dimethylaminomethyl)phenyl]-N-methylcyclohexylamin und 0.3 ml Triethylamin werden in 20 ml Methylenchlorid vorgelegt und 210 mg (1.1 mmol) Chlorameisensäure-4-chlorphenylester in wenig Methylenchlorid zugetropft. Es wird über Nacht gerührt, dann mit Methylenchlorid verdünnt, mit Wasser und gesättigter Kochsalzlösung gewaschen, getrocknet und eingedampft. Der Rückstand wird durch Säulenchromatographie (Kieselgel, Methylenchlorid/Methanol=9:1, v:v) gereinigt. Das erhaltene Produkt wird in Methylenchlorid gelöst, mit etherischer Salzsäure versetzt und das Hydrochlorid durch Zugabe von Ether gefällt. Nach Waschen mit Ether und Trocknen erhält man 276 mg (63.1 % d.Th.) der Titelverbindung als farbloses, amorphes Pulver.
R_{f}-Wert der freien Base: 0.62 (Kieselgel, Methylenchlorid/Methanol = 9:1, v:v)
¹H-NMR-Spektrum (200 MHz, DMSO-d₆), Signale bei ppm: 1.5-1.95 (m, 8H), 2.5-2.7 (s + m, 7H), 2.8-3.0 (2s, 3H), 3.9-4.1 (m, 1H), 4.2 (s, 2H), 7.1-7.5 (m, 8H)

### Analog werden erhalten:

(1) trans-O-Benzyl-N-4-[4-(dimethylaminomethyl)phenyl]-cyclohexyl-N-methylcarbamat,
   aus trans-4-[4-(Dimethylaminomethyl)phenyl]-N-methylcyclohexylamin und Chlorameisensäurebenzylester; farbloses Öl (nach Reinigung durch Säulenchromatographie an Aluminiumoxid (Essigsäureethylester/Petrolether = 3:1, v:v));
   R_{f}-Wert: 0.44 (Aluminiumoxid, Essigsäureethylester/Petrolether = 3:1)
(2) trans-O-Cyclohexyl-N-4-[4-(dimethylaminomethyl)phenyl]-cyclohexyl-N-methylcarbamat,
   aus trans-4-(4-(Dimethylaminomethyl)phenyl)-N-methylcyclohexylamin und Chlorameisencyclohexylester; Schmelzpunkt: 72°C (nach Säulenchromatographie an Aluminiumoxid (Petrolether/Essigsäureethylester = 3:1, v:v))
(3) trans-S-(4-Chlorphenyl)-N-4-[4-(dimethylaminomethyl)-phenyl]cyclohexyl-N-methyldithiocarbamat,
   aus trans-4-[4-(Dimethylaminomethyl)phenyl]-N-methylcyclohexylamin und Dithiochlorameisensäure-4-chlorphenylester (hergestellt aus 4-Chlorthiophenol und Thiophosgen); farbloses Pulver (nach Säulenchromatographie an Kieselgel (Methylenchlorid/Methanol = 9:1, v:v)); R_{f}-Wert: 0.65 (Kieselgel,Methylenchlorid/Methanol = 5:1, v:v)
(4) cis-S-(4-Chlorphenyl)-N-4-[4-(dimethylaminomethyl)-phenyl]cyclohexyl-N-methyldithiocarbamat-hydrochlorid,
   aus cis-4-(4-(Dimethylaminomethyl)phenyl]-N-methylcyclohexylamin und Dithiochlorameisensäure-4-chlorphenylester; farbloses Pulver; R_{f}-Wert der freien Base: 0.65 (Kieselgel, Methylenchlorid/Methanol = 5:1, v:v)
(5) trans-S-(4-Chlorphenyl)-N-4-[4-(dimethylaminomethyl)-phenyl]cyclohexyl-N-methylthiocarbamat-hydrochlorid,
   aus trans-4-[4-(Dimethylaminomethyl)phenyl]-N-methylcyclohexylamin und S-(4-Chlorphenyl)-chlorthioformat; farbloses Pulver; R_{f}-Wert der freien Base:0.5 (Kieselgel, Methylenchlorid/Methanol = 9:1, v:v)
(6) cis/trans-O-(4-Chlorphenyl)-N-methyl-N-4-[4-(morpholinomethyl)phenyl]cyclohexylcarbamat-hydrochlorid,
   aus cis/trans-N-Methyl-N-4-[4-(morpholinomethyl)phenyl]-cyclohexylamin und Chlorameisensäure-4-chlorphenylester; farbloses Pulver; R_{f}-Wert der freien Base: 0.35 (Aluminiumoxid, Petrolether/Essigsäureethylester = 5:1, v:v)
(7) trans-O-(4-Chlorphenyl)-N-methyl-N-4-[4-(piperidinomethyl)phenyl]cyclohexylcarbamat-hydrochlorid,
   aus trans-N-Methyl-N-4-[4-(piperidinomethyl)phenyl]cyclohexylamin und Chlorameisensäure-4-chlorphenylester; farbloses Pulver; R_{f}-Wert der freien Base: 0.48 (Aluminiumoxid, Petrolether/Essigsäureethylester = 10:1, v:v)
(8) trans-O-(4-Chlorphenyl)-N-methyl-N-4-[4-(pyrrolidinomethyl)phenyl]cyclohexylcarbamat-hydrochlorid,
   aus trans-N-Methyl-N-4-[4-(pyrrolidinomethyl)phenyl]cyclohexylamin und Chlorameisensäure-4-chlorphenylester; farbloses Pulver; R_{f}-Wert der freien Base: 0.32 (Aluminiumoxid., Petrolether/Essigsäureethylester = 5:1, v:v)
(9) trans-O-(4-Chlorphenyl)-N-4-[4-(diethylaminomethyl)phenyl]cyclohexyl-N-methylcarbamat-hydrochlorid,
   aus trans-N-4-[4-(diethylaminomethyl)phenyl]-N-methylcyclohexylamin und Chlorameisensäure-4-chlorphenylester; farbloses Pulver; R_{f}-Wert der freien Base.0.48 (Aluminiumoxid, Petrolether/Essigsäureethylester = 5:1, v:v)
(10) trans-O-(4-Chlorphenyl)-N-4-[4-[(2-hydroxyethyl)-methylaminomethyl]phenyl]cyclohexyl-N-methylcarbamat-hydrochlorid, aus trans-N-4-[4-[(2-hydroxyethyl)methylaminomethyl]phenyl]-N-methylcyclohexylamin und Chlorameisensäure-4-chlorphenylester; farbloses Pulver; R_{f}-Wert der freien Base:0.19 (Aluminiumoxid, Petrolether/Essigsäureethylester = 1:1, v:v)
(11) trans-(O-4-Chlorphenyl)-N-4-[4-(di-n-hexylaminomethyl)phenyl]cyclohexyl-N-methylcarbamat-hydrochlorid,
   aus trans-N-4-[4-(Di-n-hexylaminomethyl)phenyl]-N-methylcyclohexylamin und Chlorameisensäure 4-chlorphenylester; farbloses Pulver; R_{f}-Wert der freien Base: 0.58 (Aluminiumoxid, Petrolether/Essigsäureethylester = 10:1, v:v)
(12) trans-N-4-[4-(Di-sec-butylaminomethyl)phenyl]cyclohexyl-O-(4-chlorphenyl)-N-methylcarbamat-hydrochlorid,
   aus trans-N-4-[4-(Di-sec-butylaminomethyl)phenyl]-N-methylcyclohexylamin und Chlorameisensäure-4-chlorphenylester; farbloses Pulver; R_{f}-Wert der freien Base: 0.60 (Aluminiumoxid, Petrolether/Essigsäureethylester = 10:1, v:v)
(13) trans-O-(4-Chlorphenyl)-N-methyl-N-4-[4-(N-methyl-N-allylaminomethyl)phenyl]cyclohexylcarbamat,
   aus trans-N-Methyl-N-4-[4-(N-methyl-N-allylaminomethyl)-phenyl]cyclohexylamin und Chlorameisensäure-4-chlorphenylester; farbloses Pulver; R_{f}-Wert: 0.58 (Petrolether/Essigsäureethylester = 5:1, v:v)
(14) trans-O-(4-Chlorphenyl)-N-methyl-N-4-[4-(N-methyl-N-propargylaminomethyl)phenyl]cyclohexylcarbamat,
   aus trans-N-Methyl-N-4-[4-(N-methyl-N-propargylaminomethyl)-phenyl]cyclohexylamin und Chlorameisensäure-4-chlorphenylester; farbloses Pulver; R_{f}-Wert: 0.38 (Aluminiumoxid, Petrolether/Essigsäureethylester = 5:1, v:v)
(15) trans-O-(4-Chlorphenyl)-N-methyl-N-4-[4-(methylaminomethyl)phenyl]cyclohexylcarbamat-hydrochlorid,
   aus trans-N-4-[4-(tert.-Butyloxycarbonylmethylaminomethyl)-phenyl]-N-methylcyclohexylamin und Chlorameisensäure-4-chlorphenylester, gefolgt von Abspaltung der tert.-Butyloxycarbonylgruppe mit etherischer Salzsäure; farbloses Pulver; R_{f}-Wert der freien Base: 0.5 (Aluminiumoxid, Methylenchlorid/Methanol = 40:1,.v:v)
(16) trans-N-4-[4-(Dimethylaminomethyl)phenyl]cyclohexyl-N-methyl-O-(4-methylphenyl)carbamat,
   aus N-4-[4-(Dimethylaminomethyl)phenyl)-N-methylcyclohexylamin und Chlorameisensäure-4-methylphenylester;
   R_{f}-Wert der freien Base: 0.47 (Aluminiumoxid, Petrolether/Essigsäureethylester = 5:1, v:v);
   Schmelzpunkt der freien Base: 93°C.
   Durch Behandeln mit Salzsäure wurde das Hydrochlorid erhalten; farbloses Pulver vom Schmelzpunkt 260°C.
   Durch Behandeln der freien Base mit Methansulfonsäure in einem Essigsäureethylester-Ether-Gemisch wurde das Methansulfonat erhalten. Farbloses Pulver vom Schmelzpunkt 165°C.
   Durch Behandeln der freien Base mit L-Weinsäure in einem Methanol-Essigsäureethylester-Gemisch wurde das Tartrat erhalten. Farbloses Pulver vom Schmelzpunkt 135°C.
(17) trans-N-Methyl-O-(4-methylphenyl)-N-4-[4-(piperidinomethyl)phenyl]cyclohexylthiocarbamat-hydrochlorid,
   aus trans-N-Methyl-N-4-[4-(piperidinomethyl)phenyl]cyclohexylamin und Chlorthioameisensäure-O-4-methylphenylester; farbloses Pulver; R_{f}-Wert der freien Base: 0.35 (Aluminiumoxid, Petrolether/Essigsäureethylester = 10:1, v:v)
(18) trans-N-Methyl-O-(4-methylphenyl)-N-4-[4-(piperidinomethyl)phenyl]cyclohexylcarbamat-hydrochlorid,
   aus trans-N-Methyl-N-4-[4-(piperidinomethyl)phenyl]cyclohexylamin und Chlorameisensäure-4-methylphenylester; farbloses Pulver; R_{f}-Wert der freien Base: 0.43 (Aluminiumoxid, Petrolether/Essigsäureethylester = 10:1, v:v)
(19) trans-N-4-[4-(Dimethylaminomethyl)phenyl]cyclohexyl-N-methyl-O-(4-methylphenyl)thiocarbamat-hydrochlorid,
   aus trans-N-4-[4-(Dimethylaminomethyl)phenyl]-N-methylcyclohexylamin und Chlorthioameisensäure-O-4-methylphenylester; farbloses Pulver; R_{f}-Wert der freien Base: 0.29 (Aluminiumoxid, Petrolether/Essigsäureethylester = 5:1, v:v)
(20) trans-N-4-[4-(Dimethylaminomethyl)phenyl]cyclohexyl-O-4-fluorphenyl-N-methylcarbamat,
   aus trans N-4-[4-(Dimethylaminomethyl)phenyl]-N-methylcyclohexylamin und Chlorameisensäure-O-4-fluorphenylester;
   R_{f}-Wert der freien Base: 0.34 (Aluminiumoxid, Petrolether/Essigsäureethylester = 5:1, v:v);
   Schmelzpunkt der freien Base 85°C.
   Durch Behandeln mit Salzsäure wurde das Hydrochlorid erhalten; farbloses Pulver vom Schmelzpunkt 250°C.
   Durch Behandeln der freien Base mit Methansulfonsäure in einem Essigsäureethylester-Ether-Gemisch wurde das Methansulfonat erhalten. Farbloses Pulver vom Schmelzpunkt 190°C.
   Durch Behandeln der freien Base mit L-Weinsäure in einem Methanol-Essigsäureethylester-Gemisch wurde das Tartrat erhalten. Farbloses Pulver vom Schmelzpunkt 165°C.
(21) trans-O-(4-Fluorphenyl)-N-methyl-N-4-[4-(piperidinomethyl)phenyl]cyclohexylcarbamat-hydrochlorid,
   aus N-Methyl-N-4-[4-(piperidinomethyl)phenyl]cyclohexylamin und Chlorameisensäure-4-fluorphenylester; farbloses Pulver;
   R_{f}-Wert der freien Base: 0.49 (Aluminiumoxid, Petrolether/Essigsäureethylester = 5:1, v:v)
(22) trans-N-Methyl-O-phenyl-N-4-[4-(piperidinomethyl)phenyl]-cyclohexylcarbamat-hydrochlorid,
   aus trans N-Methyl-N-4-[4-(piperidinomethyl)phenyl]cyclohexylamin und Chlorameisensäurephenylester; farbloses Pulver; Schmelzpunkt: 218-223°C.

### Beispiel 2

### trans-O-(4-Chlorphenyl)-N-4-[4-(dimethylaminomethyl)phenyl]-cyclohexyl-N-methylthiocarbamat-hydrochlorid

Eine Lösung von 8.6 g (35 mmol) trans-4-[4-(Dimethylaminomethyl)phenyl]-N-methylcyclohexylamin in 100 ml Chloroform wird unter Eiskühlung mit 35 ml lN-Natronlauge und 70 ml Wasser versetzt. Unter starkem Rühren werden unter Eiskühlung 7.4 g Chlorthioameisensäure-O-4-chlorphenylester in 50 ml Chloroform innerhalb von 20 Minuten zugetropft und 30 Minuten bei 0°C und anschliessend 1 Stunde bei Raumtemperatur gerührt. Die organische Phase wird abgetrennt, mit Wasser gewaschen, mit Magnesiumsulfat getrocknet und eingedampft. Das nach Reinigung durch Säulenchromatographie (Kieselgel, Methylenchlorid/Methanol = 5:1, v:v) erhaltene Produkt wird in 50 ml Methylenchlorid gelöst und mit etherischer Salzsäure und dann Ether versetzt.
Ausbeute: 11.3 g (71.2 % d.Th.) der Titelverbindung; farbloses Pulver; Schmelzpunkt: 257-259°C (Zers.).
¹H-NMR-Spektrum (200 MHz, DMSO-d₆); Signale bei ppm:
1.5-2.0 (m, 8 H), 2.55-2.7 (s+m, 6+1H), 3.3-3.4 (d, 3H), 4.2 (s, 2H), 4.6 und 4.9 (2m, 1H), 7.1-7.5 (m, 8H)

### Beispiel 3

### trans-S-Benzyl-N-4-[4-(dimethylaminomethyl)phenyl]cyclohexyl-N-methyldithiocarbamat

Zu 500 mg (2mmol) trans-N-4-[4-(Dimethylaminomethyl)phenyl]-N-methylcyclohexylamin, gelöst in 12ml Tetrahydrofuran, werden bei -15 bis -10°C 1.25 ml einer 6-molaren Lösung von n-Butyllithium in n-Hexan getropft.Es wird 1 Stunde bei -15°C nachgerührt und dann 182 mg (2.4 mmol) Schwefelkohlenstoff in 1 ml Tetrahydrofuran zugegeben. Nach einer Stunde bei -10°C werden 340 mg (2mmol) Benzylbromid in 2ml Tetrahydrofuran bei 0°C zugetropft und anschliessend über Nacht bei Raumtemperatur gerührt. Nach Zugabe von Wasser wird mit Ether extrahiert, die organische Phase mit Magnesiumsulfat getrocknet und eingedampft. Nach Reinigung durch Säulenchromatographie (Aluminiumoxid, Petrolether/Essigsäureethylester = 5:1, v:v) erhält man 280 mg (33.9 % d. Th.) der Titelverbindung als farblose Kristalle.
R_{f}-Wert: 0.46 (Aluminiumoxid, Petrolether/Essigsäureethylester = 5:1, v:v).
¹H-NMR-Spektrum (200MHz,DMSO-d₆); Signale bei ppm:
1.4-2.0 (m, 8H), 2.1 (s, 6H), 2.5 (m, 1H), 3.2-3.4 (3s, 5H), 4.5+5.5 (2m, 1H), 4.5 (s, 2H), 7.2-7.5 (m, 9H)

### Analog werden erhalten:

(1) trans-S-Cyclohexylmethyl-N-4-[4-(dimethylaminomethyl)-phenyl]cyclohexyl-N-methyldithiocarbamat,
   aus trans-N-4-[4-(Dimethylaminomethyl)phenyl]-N-methylcyclohexylamin und (Brommethyl)-cyclohexan; farblose Kristalle;
   R_{f}-Wert: 0.5 (Aluminiumoxid, Petrolether/Essigsäure-ethylester = 5:1, v:v)
(2) trans-S-Cyclohexyl-N-4-[4-(dimethylaminomethyl)phenyl]-cyclohexyl-N-methyldithiocarbamat,
   aus trans-N-4-[4-(Dimethylaminomethyl)phenyl]-N-methylcyclohexylamin und Cyclohexylbromid; farblose Kristalle;
   R_{f}-Wert: 0.53 (Aluminiumoxid, Petrolether/Essigsäureethylester = 5:1, v:v)
(3) trans-S-(n-Butyl)-N-4-[4-(dimethylaminomethyl)phenyl]-cyclohexyl-N-methyldithiocarbamat-hydrochlorid,
   aus trans-N-4-[4-(Dimethylaminomethyl)phenyl]-N-methylcyclohexylamin und n-Butylbromid; farbloses Pulver;
   R_{f}-Wert der freien Base: 0.48 (Aluminiumoxid, Petrolether/Essigsäureethylester = 5:1)
(4) trans-N-4-[4-(Dimethylaminomethyl)phenyl]cyclohexyl-S-(2-fluorbenzyl)-N-methyldithiocarbamat-hydrochlorid,
   aus trans-N-4-[4-(Dimethylaminomethyl)phenyl]-N-methylcyclohexylamin und 2-Fluorbenzylchlorid; farbloses Pulver;
   R_{f}-Wert der freien Base: 0.63 (Kieselgel, Methylenchlorid/Methanol = 5:1, v:v)
(5) trans-N-4-[4-(Dimethylaminomethyl)phenyl]cyclohexyl-S-3-fluorbenzyl-N-methyldithiocarbamat-hydrochlorid,
   aus trans-N-4-[4-(Dimethylaminomethyl)phenyl]-N-methylcyclohexylamin und 3-Fluorbenzylchlorid; farbloses Pulver;
   R_{f}-Wert der freien Base: 0.57 (Kieselgel, Methylenchlorid/Methanol = 5:1, v:v)
(6) trans-N-4-[4-(Dimethylaminomethyl)phenyl]cyclohexyl-S-(4-fluorbenzyl)-N-methyldithiocarbamat-hydrochlorid,
   aus trans-N-4-[4-(Dimethylaminomethyl)phenyl]-N-methylcyclohexylamin und 4-Fluorbenzylchlorid; farbloses Pulver;
   R_{f}-Wert der freien Base: 0.57 (Kieselgel, Methylenchlorid/Methanol = 5:1, v:v).
(7) trans-S-(4-Chlorbenzyl)-N-4-[4-(dimethylaminomethyl)-phenyl]cyclohexyl-N-methyldithiocarbamat-hydrochlorid,
   aus trans-N-4-[4-(Dimethylaminomethyl)phenyll-N-methylcyclohexylamin und 4-Chlorbenzylchlorid; farbloses Pulver;
   R_{f}-Wert der freien Base: 0.48 (Kieselgel, Methylenchlorid/Methanol = 5:1, v:v)
(8) trans-N-4-[4-(Dimethylaminomethyl)phenyl]cyclohexyl-N-methyl-S-(1-phenethyl)dithiocarbamat-hydrochlorid,
   aus trans-N-4-[4-(Dimethylaminomethyl)phenyl]-N-methylcyclohexylamin und 1-Phenethylchlorid; farbloses Pulver;
   R_{f}-Wert der freien Base : 0.60 (Kieselgel, Methylenchlorid/Methanol = 5:1, v:v)
(9) cis-S-Benzyl-N-4-(4-(dimethylaminomethyl)phenyl]-cyclohexyl-N-methyldithiocarbamat-hydrochlorid,
   aus cis-N-4-[4-(Dimethylaminomethyl)phenyl]-N-methylcyclohexylamin und Benzylbromid; farbloses Pulver; R_{f}-Wert der freien Base: 0.51 (Kieselgel.Methylenchlorid/Methanol=5:1, v:v)
(10) trans-S-Benzyl-N-4-[4-(dimethylaminomethyl)phenyl]cyclohexyl-N-isopropyldithiocarbamat-hydrochlorid,
   aus trans-N-4-[4-(Dimethylaminomethyl)phenyl]-N-isopropylcyclohexylamin und Benzylbromid; farbloses Pulver;
   R_{f}-Wert der freien Base. 0.58 (Kieselgel, Methylenchlorid/Methanol = 5:1, v:v)
(11) trans-S-Benzyl-N-4-[4-(dimethylaminomethyl)phenyl]cyclohexyl-N-(n-hexyl)dithiocarbamat-hydrochlorid,
   aus trans-N-4-[4-(Dimethylaminomethyl)phenyl]-N-(n-hexyl)-cyclohexylamin und Benzylbromid; farbloses Pulver;
   R_{f}-Wert der freien Base: 0.63 (Kieselgel, Methylenchlorid/Methanol = 5:1, v:v).
(12) trans-N-Allyl-S-benzyl-N-4-[4-(dimethylaminomethyl)-phenyl)cyclohexyldithiocarbamat-hydrochlorid,
   aus trans-N-Allyl-N-4-[4-(dimethylaminomethyl)phenyl]cyclohexylamin und Benzylbromid; farbloses Pulver;
   R_{f}-Wert der freien Base: 0.60 (Kieselgel, Methylenchlorid/Methanol = 5:1,v:v).

## Patentansprüche

1. Urethane und deren Thio- und Dithioanalogen der allgemeinen Formel 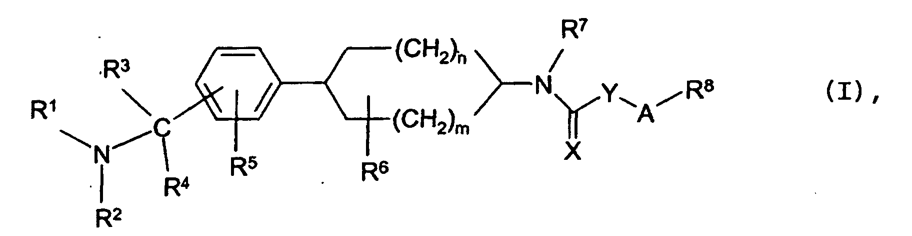 in der
m die Zahlen 0 oder 1,
n die Zahlen 1 oder 2,
A eine Einfachbindung, eine geradkettige oder verzweigte C₁₋₈-Alkylengruppe, eine C₂₋₈-Alkenylen- oder C₂₋₈-Alkinylengruppe, wobei eine ungesättigte Gruppe nicht direkt an den Rest Y gebunden ist,
X ein Sauerstoff- oder Schwefelatom,
Y ein Sauerstoff- oder Schwefelatom,
R¹ eine geradkettige oder verzweigte C₁₋₈-Alkylgruppe, eine C₁₋₆-Alkenylgruppe oder eine C₁₋₆-Alkinylgruppe, wobei die Mehrfachbindung von der Stickstoff-Kohlenstoff-Bindung isoliert ist,
R² ein Wasserstoffatom, eine geradkettige oder verzweigte C₁₋₈-Alkylgruppe, die durch eine Hydroxy- oder Alkoxygruppe substituiert sein kann, wobei ein Hydroxy- und Alkoxy-Substituent nicht in 1-Stellung gebunden ist, eine C₁₋₆-Alkenylgruppe oder eine C₁₋₆-Alkinylgruppe, wobei eine Mehrfachbindung von der Stickstoff-Kohlenstoff-Bindung isoliert ist, oder
R¹ und R² zusammen mit dem Stickstoffatom einen 5- bis 7-gliedrigen, gesättigten heterocyclischen Ring, in dem eine von dem Stickstoffatom isolierte Methylengruppe durch ein Sauerstoff- oder Schwefelatom, durch eine -NH- oder -N(Alkyl)-Gruppe ersetzt sein kann,
R³ bis R⁶, die gleich oder verschieden sein können, Wasserstoffatome oder Alkylgruppen,
R⁷ eine geradkettige oder verzweigte C₁₋₆-Alkylgruppe, eine C₁₋₆-Alkenylgruppe oder eine C₁₋₆-Alkinylgruppe, wobei die Mehrfachbindung von der Stickstoff-Kohlenstoff-Bindung isoliert ist, und
R⁸ eine C₃₋₇-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe oder, sofern A keine Einfachbindung darstellt, auch ein Wasserstoffatom bedeuten,
wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
mit der Maßgabe, daß trans-N-tert.Butoxycarbonyl-N-methyl-4-[4-(3-hydroxypropyl)methylaminomethylphenyl]cyclohexylamin ausgenommen wird, deren Enantiomere, Diastereomere, deren Gemische und deren Salze.

2. Verbindungen der allgemeinen Formel I gemäß Anspruch 1, in der
m die Zahl 1,
n die Zahl 1,
A eine Einfachbindung, eine geradkettige oder verzweigte C₁₋₆-Alkylengruppe,
X ein Sauerstoff- oder Schwefelatom,
Y ein Sauerstoff- oder Schwefelatom,
R¹ eine geradkettige oder verzweigte C₁₋₆-Alkylgruppe,
R² ein Wasserstoffatom, eine geradkettige oder verzweigte C₁₋₆-Alkylgruppe, die durch eine Hydroxygruppe substituiert sein kann, wobei die Hydroxygruppe nicht in 1-Stellung gebunden ist, eine C₁₋₄-Alkenylgruppe oder eine C₁₋₄-Alkinylgruppe, wobei die Mehrfachbindung von der Stickstoff-Kohlenstoff-Bindung isoliert ist, oder
R¹ und R² zusammen mit dem Stickstoffatom einen 5-bis 7-gliedrigen, gesättigten heterocyclischen Ring, in dem eine von dem Stickstoffatom isolierte Methylengruppe durch ein Sauerstoffatom ersetzt sein kann,
R³ bis R⁶, die gleich oder verschieden sein können, Wasserstoffatome oder Methylgruppen,
R⁷ eine geradkettige oder verzweigte C₁₋₆-Alkylgruppe oder eine C₁₋₄-Alkenylgruppe, wobei die Mehrfachbindung von der Stickstoff-Kohlenstoff-Bindung isoliert ist, und
R⁸ eine C₃₋₆-Cycloalkylgruppe, eine gegebenenfalls durch ein oder zwei Halogenatome, durch eine Alkyl-, Alkoxy-, Trifluormethyl- oder Cyanogruppe substituierte Phenyl- oder Naphtylgruppe oder, sofern A keine Einfachbindung darstellt, auch ein Wasserstoffatom bedeuten,
wobei, sofern nichts anderes erwähnt wurde, in den vorstehend erwähnten Resten enthaltene Alkylgruppen jeweils 1 bis 3 Kohlenstoffatome enthalten können und ein vorstehend erwähntes Halogenatom ein Fluor-, Chlor- oder Bromatom bedeuten kann,
mit der Maßgabe, daß trans-N-tert.Butoxycarbonyl-N-methyl-4-[4-(3-hydroxypropyl)methylaminomethylphenyl]cyclohexylamin ausgenommen wird, deren Enantiomere, Diastereomere, deren Gemische und deren Salze.

3. Verbindungen der allgemeinen Formel I gemäß Anspruch 1, in der
m die Zahl 1,
n die Zahl 1,
A eine Einfachbindung oder eine Methylengruppe,
X ein Sauerstoff- oder Schwefelatom,
Y ein Sauerstoff- oder Schwefelatom,
R¹ eine Methyl- oder Ethylgruppe,
R² eine Methyl-, Ethyl-, Allyl- oder Propargylgruppe oder
R¹ und R² zusammen mit dem Stickstoffatom einen Pyrrolidin- oder Piperidinring,
R³ bis R⁶ Wasserstoffatome,
R⁷ eine Methylgruppe und
R⁸ eine gegebenenfalls durch ein Chlor- oder Fluoratom oder durch eine Methylgruppe substituierte Phenylgruppe bedeuten, deren Gemische und deren Salze.

4. Folgende Verbindungen der allgemeinen Formel I gemäß Anspruch 1:
(1) trans-S-(4-Chlorphenyl)-N-4-[4-(dimethylaminomethyl)-phenyl]cyclohexyl-N-methyldithiocarbamat,
(2) trans-S-(3-Fluorbenzyl)-N-4-[4-(dimethylaminomethyl)-phenyl]cyclohexyl-N-methyldithiocarbamat,
(3) trans-S-(4-Fluorbenzyl)-N-4-[4-(dimethylaminomethyl)-phenyl]cyclohexyl-N-methyldithiocarbamat,
(4) trans-S-(4-Chlorbenzyl)-N-4-[4-(dimethylaminomethyl)-phenyl]cyclohexyl-N-methyldithiocarbamat,
(5) trans-O-(4-Chlorphenyl)-N-4-[4-(dimethylaminomethyl)-phenyl]cyclohexyl-N-methylcarbamat,
(6) cis-S-Benzyl-N-4-[4-(dimethylaminomethyl)phenyl]-cyclohexyl-N-methyldithiocarbamat,
(7) trans-O-(4-Chlorphenyl)-N-4-[4-(dimethylaminomethyl)-phenyl]cyclohexyl-N-methylthiocarbamat,
(8) trans-S-(4-Chlorphenyl)-N-4-[4-(dimethylaminomethyl)-phenyl]cyclohexyl-N-methylthiocarbamat,
(9) trans-O-(4-Chlorphenyl)-N-methyl-N-4-[4-(piperidinomethyl)phenyl]cyclohexylcarbamat,
(10) trans-O-(4-Chlorphenyl)-N-methyl-N-4-(4-(pyrrolidinomethyl)phenyl]cyclohexylcarbamat,
(11) trans-O-(4-Chlorphenyl)-N-4-[4-(diethylaminomethyl)-phenyl]cyclohexyl-N-methylcarbamat,
(12) trans-O-(4-Chlorphenyl)-N-methyl-N-4-[4-(N-methyl-N-allylaminomethyl)phenyl]cyclohexylcarbamat,
(13) trans-O-(4-Chlorphenyl)-N-methyl-N-4-[4-(N-methyl-N-propargylaminomethyl)phenyl]cyclohexylcarbamat,
(14) trans-N-4-[4-(Dimethylaminomethyl)phenyl]cyclohexyl-N-methyl-O-(4-methylphenyl)carbamat,
(15) trans-N-4-[4-(Dimethylaminomethyl)phenyl]cyclohexyl-N-methyl-O-(4-methylphenyl)thiocarbamat,
(16) trans-N-Methyl-O-(4-methylphenyl)-N-4-[4-(piperidinomethyl)phenyl]cyclohexylthiocarbamat und
(17) trans-N-4-[4-(Dimethylaminomethyl)phenyl]cyclohexyl-O-(4-fluorphenyl)-N-methylcarbamat,
deren Gemische und deren Salze.

5. Phystologisch verträgliche Salze der Verbindungen nach mindestens einem der Ansprüche 1 bis 4 mit anorganischen oder organischen Säuren.

6. Arzneimittel, enthaltend eine Verbindung nach mindestens einem der Ansprüche 1 bis 4 oder ein physiologisch verträgliches Salz gemäß Anspruch 5 neben gegebenenfalls einem oder mehreren inerten Trägerstoffen und/oder Verdünnungsmitteln.

7. Arzneimittel gemäß Anspruch 6, geeignet zur Behandlung von Krankheiten, bei denen die Cholesterol-Biosynthese eine Rolle spielt.

8. Arzneimittel gemäß Anspruch 7, enthaltend eine Verbindung nach mindestens einem der Ansprüche 1 bis 4 oder ein physiologisch verträgliches Salz gemäß Anspruch 5 in Kombination mit einem oder mehreren weiteren Wirkstoffen mit cholesterol- oder lipidsenkender Aktivität.

9. Arzneimittel gemäß Anspruch 8, **dadurch gekennzeichnet, daß** die weiteren Wirkstoffe ausgewählt sind aus
gallensäurebindenden Harzen,
Verbindungen, die die Cholesterolresorption hemmen,
Verbindungen, die über einen anderen Mechanismus als Hemmung der 2,3-Epoxisqualen-Lanosterol-Cyclase in die Cholesterolbiosynthese eingreifen,
Fibraten, Nikotinsäure, deren Derivate und Analoge, sowie Probucol.

10. Arzneimittel gemäß Anspruch 7, 8 oder 9, geeignet zur Behandlung oder Prophylaxe der Hypercholesterolämie, der Hyperlipoproteinämie, der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens oder Gangrän, zur Behandlung von Erkrankungen, die mit überhöhter Zellproliferation im Zusammenhang stehen, zur Prophylaxe und Behandlung von Gallensteinleiden oder zur Behandlung von Mykosen.

11. Verwendung einer Verbindung nach mindestens einem der Ansprüche 1 bis 5 zur Herstellung eines Arzneimittels gemäß einem der Ansprüche 7 bis 10.

12. Futtermittel für Legehennen, enthaltend eine Verbindung nach mindestens einem der Ansprüche 1 bis 4 oder ein physiologisch verträgliches Salz gemäß Anspruch 5.

13. Verwendung einer Verbindung nach mindestens einem der Ansprüche 1 bis 5 zur Herstellung eines Futtermittels für Legehennen zur Erzeugung cholesterolarmer Eier.

14. Verfahren zur Herstellung eines Arzneimittels gemäß Anspruch 6, **dadurch gekennzeichnet, daß** auf nichtchemischem Wege eine Verbindung nach mindestens einem der Ansprüche 1 bis 5 in einen oder mehrere inerte Trägerstoffe und/oder Verdünnungsmittel eingearbeitet wird.

15. Verfahren zur Herstellung der Verbindungen nach mindestens einem der Ansprüche 1 bis 5, **dadurch gekennzeichnet, daß**
a) eine Verbindung der allgemeinen Formel 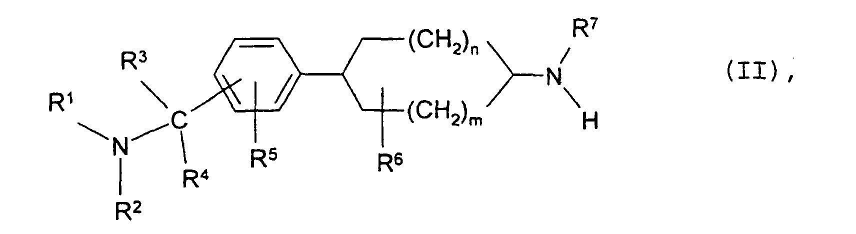 in der
m ,n und R¹ bis R⁷ wie in den Ansprüchen 1 bis 4 definiert sind, mit einer Verbindung der allgemeinen Formel 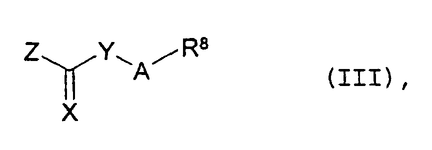 in der
A , X, Y und R⁸ wie in den Ansprüchen 1 bis 4 definiert sind und Z eine Austrittsgruppe bedeutet,
umgesetzt wird und gegebenenfalls verwendete Schutzgruppen anschließend abspalten werden oder
b) zur Herstellung von Verbindungen der allgemeinen Formel (I), in der X und Y jeweils ein Schwefelatom bedeuten und m, n, A und R¹ bis R⁸ mit der Maßgabe wie in den Ansprüchen 1 bis 4 definiert sind, daß R⁸ keine gegebenenfalls substituierte Phenyl- oder Naphtylgruppe darstellt, falls A eine Einfachbindung bedeutet, eine Verbindung der allgemeinen Formel (II), in der m, n und R¹ bis R⁷ wie in den Ansprüchen 1 bis 4 definiert sind, mit Schwefelkohlenstoff und anschließend mit einem Alkylierungsmittel der allgemeinen Formel 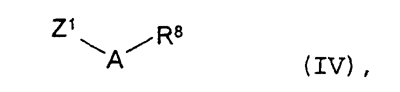 in der
A und R⁸ mit der Maßgabe wie in den Ansprüchen 1 bis 4 definiert sind, daß R⁸ keine gegebenenfalls substituierte Phenyloder Naphtylgruppe darstellt, falls A eine Einfachbindung bedeutet, und Z¹ eine Austrittsgruppe bedeutet, umgesetzt wird und gegebenenfalls verwendete Schutzgruppen anschließend abspalten werden und
gewünschtenfalls ein so erhaltenes Gemisch der geometrischen Isomeren einer Verbindung der allgemeinen Formel I in ihre Enantiomeren und Diastereomeren aufgetrennt wird oder
eine so erhaltene Verbindung der allgemeinen Formel I in ihr Salz mit einer anorganischen oder organischen Säure, insbesondere in ihre physiologisch verträglichen Salze, übergeführt wird.

## Claims

1. Urethanes and the thio and dithio analogues thereof of the general formula 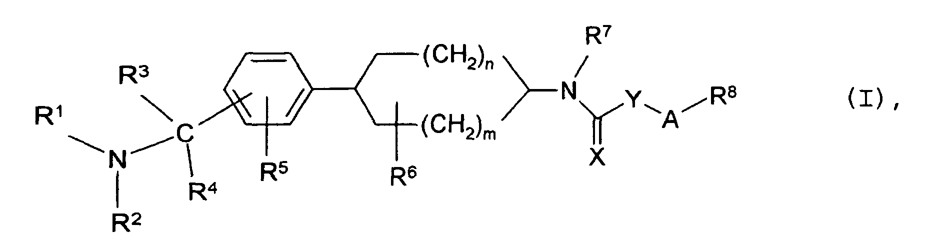 wherein
m denotes the numbers 0 or 1,
n denotes the numbers 1 or 2,
A denotes a single bond, a straight-chained or branched C₁₋₈-alkylene group, a C₂₋₈-alkenylene or C₂₋₈-alkynylene group, whilst an unsaturated group is not directly bound to the group Y,
X denotes an oxygen or sulphur atom,
Y denotes an oxygen or sulphur atom,
R¹ denotes a straight-chained or branched C₁₋₈-alkyl group, a C₁₋₆-alkenyl group or a C₁₋₆-alkynyl group, whilst the multiple bond is isolated from the nitrogen-carbon bond,
R² denotes a hydrogen atom, a straight-chained or branched C₁₋₈-alkyl group which may be substituted by a hydroxy or alkoxy group, whilst a hydroxy and alkoxy substituent is not bound in the 1-position, a C₁₋₆-alkenyl group or a C₁₋₆-alkynyl group, whilst a multiple bond is isolated from the nitrogen-carbon bond, or
R¹ and R² together with the nitrogen atom denote a 5- to 7-membered, saturated heterocyclic ring wherein a methylene group isolated from the nitrogen atom may be replaced by an oxygen or sulphur atom or by an -NH- or -N(alkyl)- group,
R³ to R⁶, which may be identical or different, denote hydrogen atoms or alkyl groups,
R⁷ denotes a straight-chained or branched C₁₋₆-alkyl group, a C₁₋₆-alkenyl group or a C₁₋₆-alkynyl group, whilst the multiple bond is isolated from the nitrogen-carbon bond,
R⁸ denotes a C₃₋₇-cycloalkyl group, a phenyl or naphthyl group optionally substituted by one or two halogen atoms or by an alkyl, alkoxy, tri fluoromethyl or cyano group or, if A does not denote a single bond, R⁸ also denotes a hydrogen atom,
whilst, unless otherwise stated, alkyl groups contained in the groups mentioned above may contain 1 to 3 carbon atoms and a halogen atom mentioned'above may be a fluorine, chlorine or bromine atom,
with the proviso that trans-N-tert.butoxycarbonyl-N-methyl-4-[4-(3-hydroxypropyl)methylaminomethylphenyl]cyclohexylamine is excepted,
the enantiomers, diastereomers, the mixtures thereof and the salts thereof.

2. Compounds of general formula I according to claim 1, wherein
m denotes the number 1,
n denotes the number 1,
A denotes a single bond, a straight-chained or branched C₁₋₆-alkylene group,
X denotes an oxygen or sulphur atom,
Y denotes an oxygen or sulphur atom,
R¹ denotes a straight-chained or branched C₁₋₆-alkyl group,
R² denotes a hydrogen atom, a straight-chained or branched C₁₋₆-alkyl group which may be substituted by a hydroxy group, whilst the hydroxy group is not bound in the 1 position, a C₁₋ ₄-alkenyl group or a C₁₋₄-alkynyl group, whilst the multiple bond is isolated from the nitrogen-carbon bond, or
R¹ and R² together with the nitrogen atom denote a 5- to 7-membered, saturated heterocyclic ring wherein a methylene group isolated from the nitrogen atom may be replaced by an oxygen atom,
R³ to R⁶, which may be identical or different, denote hydrogen atoms or methyl groups,
R⁷ denotes a straight-chained or branched C₁₋₆-alkyl group or a C₁₋₄-alkenyl group, whilst the multiple bond is isolated from the nitrogen-carbon bond, and
R⁸ denotes a C₃₋₆-cycloalkyl group, a phenyl or naphthyl group optionally substituted by one or two halogen atoms or by an alkyl, alkoxy, tri fluoromethyl or cyano group or, if A does not denote a single bond, R⁸ also denotes a hydrogen atom,
whilst, unless otherwise stated, alkyl groups contained in the groups mentioned above may each contain 1 to 3 carbon atoms and a halogen atom mentioned above may be a fluorine, chlorine or bromine atom,
with the proviso that trans-N-tert.butoxycarbonyl-N-methyl-4-[4-(3-hydroxypropyl)methylaminomethylphenyl]cyclohexylamine is excepted,
the enantiomers, diastereomers, the mixtures thereof and the salts thereof.

3. Compounds of general formula I according to claim 1, wherein
m denotes the number 1,
n denotes the number 1,
A denotes a single bond or a methylene group,
X denotes an oxygen or sulphur atom,
Y denotes an oxygen or sulphur atom,
R¹ denotes a methyl or ethyl group,
R² denotes a methyl, ethyl, allyl or propargyl group or
R¹ and R² together with the nitrogen atom denote a pyrrolidine or piperidine ring,
R³ to R⁶ denote hydrogen atoms,
R⁷ denotes a methyl group,
R⁸ denotes a phenyl group optionally substituted by a chlorine or fluorine atom or by a methyl group,
the mixtures and the salts thereof

4. The following compounds of general formula I according to claim 1:
(1) trans-S-(4-chlorophenyl)-N-4-[4-(dimethylaminomethyl)-phenyl]cyclohexyl-N-methyldithiocarbamate,
(2) trans-S-(3-fluorobenzyl)-N-4-[4-(dimethylaminomethyl)-phenyl]cyclohexyl-N-methyldithiocarbamate,
(3) trans-S-(4-fluorobenzyl)-N-4-[4-(dimethylaminomethyl)-phenyl]cyclohexyl-N-methyldithiocarbamate,
(4) trans-S-(4-chlorobenzyl)-N-4-[4-(dimethylaminomethyl)-phenyl]cyclohexyl-N-methyldithiocarbamate,
(5) trans-O-(4-chlorophenyl)-N-4-[4-(dimethylaminomethyl)-phenyl]cyclohexyl-N-methylcarbamate,
(6) cis-S-benzyl-N-4-[4-(dimethylaminomethyl)phenyl]-cyclohexyl-N-methyldithiocarbamate,
(7) trans-O-(4-chlorophenyl)-N-4-[4-(dimethylaminomethyl)-phenyl]cyclohexyl-N-methylthiocarbamate,
(8) trans-S-(4-chlorophenyl)-N-4-[4-(dimethylaminomethyl)-phenyl]cyclohexyl-N-methylthiocarbamate,
(9) trans-O-(4-chlorophenyl)-N-methyl-N-4-[4-(piperidinomethyl)phenyl]cyclohexylcarbamate,
(10) trans-O-(4-chlorophenyl)-N-methyl-N-4-[4-(pyrrolidinomethyl)phenyl]cyclohexylcarbamate,
(11) trans-O-(4-chlorophenyl)-N-4-[4-(diethylaminomethyl)-phenyl]cyclohexyl-N-methylcarbamate,
(12) trans-O-(4-chlorophenyl)-N-methyl-N-4-[4-(N-methyl-N-allylaminomethyl)phenyl]cyclohexylcarbamate,
(13) trans-O-(4-chlorophenyl)-N-methyl-N-4-[4-(N-methyl-N-propargylaminomethyl)phenyl]cyclohexylcarbamate,
(14) trans-N-4-[4-(dimethylaminomethyl)phenyl]cyclohexyl-N-methyl-O-(4-methylphenyl)carbamate,
(15) trans-N-4-[4-(dimethylaminomethyl)phenyl]cyclohexyl-N-methyl-O-(4-methylphenyl)thiocarbamate,
(16) trans-N-methyl-O-(4-methylphenyl)-N-4-[4-(piperidinomethyl)phenyl]cyclohexylthiocarbamate and
(17) trans-N-4-[4-(dimethylaminomethyl)phenyl]cyclohexyl-O-(4-fluorophenyl)-N-methylcarbamate,
the mixtures thereof and the salts thereof.

5. Physiologically acceptable salts of the compounds according to at least one of claims 1 to 4 with inorganic or organic acids.

6. Pharmaceutical compositions containing a compound according to at least one of claims 1 to 4 or a physiologically acceptable salt according to claim 5 optionally together with one or more inert carriers and/or diluents.

7. Pharmaceutical compositions according to claim 6, suitable for treating diseases in which cholesterol biosynthesis plays a part.

8. Pharmaceutical compositions according to claim 7, containing a compound according to at least one of claims 1 to 4 or a physiologically acceptable salt according to claim 5 in combination with one or more other active substances with a cholesterol- or lipid -lowering activity.

9. Pharmaceutical compositions according to claim 8, **characterised in that** the other active substances are selected from
resins which bind bile acid,
compounds which inhibit cholesterol absorption,
compounds which are involved in cholesterol biosynthesis by a mechanism other than the inhibition of 2,3-epoxysqualene-lanosterol-cyclase,
fibrates, nicotinic acid, the derivatives and analogues thereof, and probucol.

10. Pharmaceutical compositions according to claim 7, 8 or 9, suitable for the treatment or prophylaxis of hypercholesterolaemia, hyperlipoproteinaemia, hypertriglyceridaemia and the resulting atherosclerotic vascular changes with their consequent diseases such as coronary heart disease, cerebral ischaemia, Claudicatio intermittens or gangrene, for treating diseases connected with excessive cell proliferation, for the prophylaxis and treatment of gallstone problems or for treating mycoses.

11. Use of a compound according to at least one of claims 1 to 5 for preparing a pharmaceutical composition according to one of claims 7 to 10.

12. Feed for laying hens, containing a compound according to at least one of claims 1 to 4 or a physiologically acceptable salt according to claim 5.

13. Use of a compound according to at least one of claims 1 to 5 for preparing a feed for laying hens for producing low-cholesterol eggs.

14. Process for preparing a pharmaceutical composition according to claim 6, **characterised in that** a compound according to at least one of claims 1 to 5 is incorporated in one or more inert carriers and/or diluents by a non-chemical method.

15. Process for preparing the compounds according to at least one of claims 1 to 5, **characterised in that**
a) a compound of general formula 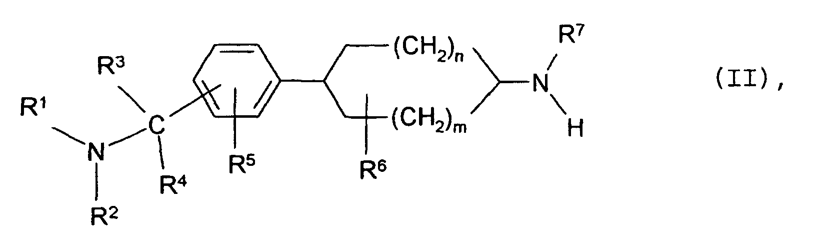 wherein
m, n and R¹ to R⁷ are defined as in claims 1 to 4, is reacted with a compound of general formula 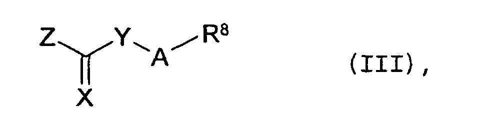 wherein
A , X, Y and R⁸ are defined as in claims 1 to 4 and Z denotes a leaving group,
and any protectng groups used are subsequently cleaved or
b) in order to prepare compounds of general formula (I) wherein X and Y each denote a sulphur atom and m, n, A and R¹ to R⁸ are defined as in claims 1 to 4, with the proviso that R⁸ does not represent an optionally substituted phenyl or naphthyl group if A denotes a single bond, a compound of general formula (II) wherein m, n and R¹ to R⁷ are defined as in claims 1 to 4, is reacted with carbon disulphide and then with an alkylating agent of general formula 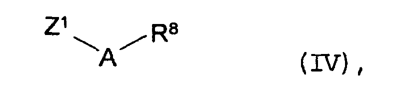
wherein
A and R⁸ are defined as in claims 1 to 4, with the proviso that R⁸ does not represent an optionally substituted phenyl or naphthyl group if A denotes a single bond, and Z¹ denotes a leaving group and any protecting groups used are subsequently cleaved and
if desired a mixture of the geometric isomers of a compound of general formula I thus obtained is resolved into its enantiomers and diastereomers or
a compound of general formula I thus obtained is converted into a salt thereof with an inorganic or organic acid, particularly into the physiologically acceptable salts thereof

## Revendications

1. Uréthanes et leurs analogues thio et dithio de formule générale 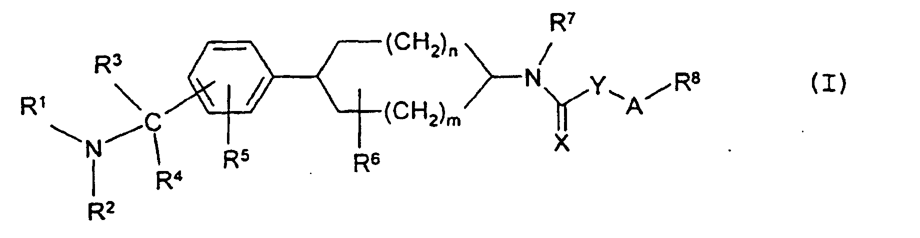 où
m représente les nombres 0 ou 1,
n représente les nombres 1 ou 2,
A représente une simple liaison, un groupe C₁₋₈-alkylène linéaire ou ramifié, un groupe C₂₋₈-alcénylène ou C₂₋₈-alcynylène, où un groupe insaturé n'est pas lié directement au groupement Y,
X représente un atome d'oxygène ou de soufre,
Y représente un atome d'oxygène ou de soufre,
R¹ représente un groupe C₁₋₈-alkyle linéaire ou ramifié, un groupe C₁₋₆-alcényle ou un groupe C₁₋₆-alcynyle, où la liaison multiple est isolée de la liaison azote-carbone,
R² représente un atome d'hydrogène, un groupe C₁₋₈-alkyle linéaire ou ramifié, qui peut être substitué par un groupe hydroxyle ou alcoxy, où un substituant hydroxyle ou alcoxy n'est pas lié en position 1, un groupe C₁₋₆-alcényle ou un groupe C₁₋₆-alcynyle, où une liaison multiple est isolée de la liaison azote-carbone, ou
R¹ et R² représentent avec l'atome d'azote un cycle hétérocyclique saturé à 5 à 7 chaînons dans lequel un groupe méthylène isolé de l'atome d'azote peut être remplacé par un atome d'oxygène ou de soufre, par un groupe -NH- ou-N(alkyle)-,
R³ à R⁶, qui peuvent être identiques ou différents, représentent des atomes d'hydrogène ou des groupes alkyle,
R⁷ représente un groupe C₁₋₆-alkyle linéaire ou ramifié, un groupe C₁₋₆-alcényle ou un groupe C₁₋₆-alcynyle, où la liaison multiple est isolée de la liaison azote-carbone, et
R⁸ représente un groupe C₃₋₇-cycloalkyle, un groupe phényle ou naphtyle éventuellement substitué par un ou deux atomes d'halogène, par un groupe alkyle, alcoxy, trifluorométhyle ou cyano, ou, dans la mesure où A ne représente pas une simple liaison, également un atome d'hydrogène,
où, sauf indication contraire, les groupes alkyle contenus dans les groupements cités précédemment peuvent contenir chacun 1 à 3 atomes de carbone et un atome d'halogène cité précédemment peut représenter un atome de fluor, de chlore ou de brome,
avec la condition que la trans-N-tert.butoxycarbonyl-N-méthyl-4-[4-(3-hydroxypropyl)méthylaminométhylphényl]cyclohexylamine est exclue,
leurs énantiomères, diastéréoisomères, leurs mélanges et leurs sels.

2. Composés de formule générale I selon la revendication 1 où
m représente le nombre 1,
n représente le nombre 1,
A représente une simple liaison, un groupe C₁₋₆-alkylène linéaire ou ramifié,
X représente un atome d'oxygène ou de soufre,
Y représente un atome d'oxygène ou de soufre,
R¹ représente un groupe C₁₋₆-alkyle linéaire ou ramifié,
R² représente un atome d'hydrogène, un groupe C₁₋₆-alkyle linéaire ou ramifié, qui peut être substitué par un groupe hydroxyle, où le groupe hydroxyle n'est pas lié en position 1, un groupe C₁₋₄-alcényle ou un groupe C₁₋₄-alcynyle, où la liaison multiple est isolée de la liaison azote-carbone, ou
R¹ et R² représentent avec l'atome d'azote un cycle hétérocyclique saturé à 5 à 7 chaînons dans lequel un groupe méthylène isolé de l'atome d'azote peut être remplacé par un atome d'oxygène,
R³ à R⁶, qui peuvent être identiques ou différents, représentent des atomes d'hydrogène ou des groupes méthyle,
R⁷ représente un groupe C₁₋₆-alkyle linéaire ou ramifié ou un groupe C₁₋₄-alcényle, où la liaison multiple est isolée de la liaison azote-carbone, et
R⁸ représente un groupe C₃₋₆-cycloalkyle, un groupe phényle ou naphtyle éventuellement substitué par un ou deux atomes d'halogène, par un groupe alkyle, alcoxy, trifluorométhyle ou cyano, ou, dans la mesure où A ne représente pas une simple liaison, également un atome d'hydrogène,
où, sauf indication contraire, les groupes alkyle contenus dans les groupements cités précédemment peuvent contenir chacun 1 à 3 atomes de carbone et un atome d'halogène cité précédemment peut représenter un atome de fluor, de chlore ou de brome,
avec la condition que la trans-N-tert.butoxycarbonyl-N-méthyl-4-[4-(3-hydroxypropyl)méthylaminométhylphényl]cyclohexylamine est exclue,
leurs énantiomères, diastéréoisomères, leurs mélanges et leurs sels.

3. Composés de formule générale I selon la revendication 1 où
m représente le nombre 1,
n représente le nombre 1,
A représente une simple liaison ou un groupe méthylène,
X représente un atome d'oxygène ou de soufre,
Y représente un atome d'oxygène ou de soufre,
R¹ représente un groupe méthyle ou éthyle,
R² représente un groupe méthyle, éthyle, allyle ou propargyle ou
R¹ et R² représentent avec l'atome d'azote un cycle pyrrolidine ou pipéridine,
R³ à R⁶ représentent des atomes d'hydrogène,
R⁷ représente un groupe méthyle et
R⁸ représente un groupe phényle éventuellement substitué par un atome de chlore
ou de fluor ou par un groupe méthyle,
leurs mélanges et leurs sels.

4. Composés de formule générale I selon la revendication 1 suivants :
(1) trans-S-(4-chlorophényl)-N-4-[4-(diméthylaminométhyl)phényl]cyclohexyl-N-méthyldithiocarbamate,
(2) trans-S-(3-fluorobenzyl)-N-4-[4-(diméthylaminométhyl)phényl]cyclohexyl-N-méthyldithiocarbamate,
(3) trans-S-(4-fluorobenzyl)-N-4-[4-(diméthylaminométhyl)phényl]cyclohexyl-N-méthyldithiocarbamate,
(4) trans-S-(4-chlorobenzyl)-N-4-[4-(diméthylaminométhyl)phényl]cyclohexyl-N-méthyldithiocarbamate,
(5) trans-O-(4-chlorophényl)-N-4-[4-(diméthylaminométhyl)phényl]cyclohexyl-N-méthylcarbamate,
(6) cis-S-benzyl-N-4-[4-(diméthylaminométhyl)phényl]cyclohexyl-N-méthyldithiocarbamate,
(7) trans-O-(4-chlorophényl)-N-4-[4-(diméthylaminométhyl)phényl]cyclohexyl-N-méthylthiocarbamate,
(8) trans-S-(4-chlorophényl)-N-4-[4-(diméthylaminométhyl)phényl]cyclohexyl-N-méthylthiocarbamate,
(9) trans-O-(4-chlorophényl)-N-méthyl-N-4- [4-(pipéridinométhyl)phényl]-cyclohexylcarbamate,
(10) trans-O-(4-chlorophényl)-N-méthyl-N-4-[4-(pyrrolidinométhyl)phényl]-cyclohexylcarbamate,
(11) trans-O-(4-chlorophényl)-N-4-[4-(diéthylaminométhyl)phényl]cyclohexyl-N-méthylcarbamate,
(12) trans-O-(4-chlorophényl)-N-méthyl-N-4-[4-(N-méthyl-N-allylaminométhyl)-phényl]cyclohexylcarbamate,
(13) trans-O-(4-chlorophényl)-N-méthyl-N-4-[4-(N-méthyl-N-propargylaminométhyl)phényl]cyclohexylcarbamate,
(14) trans-N-4-[4-(dirnéthylaminométhyl)phényl]cyclohexyl-N-méthyl-O-(4-méthylphényl)carbamate,
(15) trans-N-4-[4-(diméthylaminométhyl)phényl]cyclohexyl-N-méthyl-O-(4-méthylphényl)thiocarbamate,
(16) trans-N-méthyl-O-(4-méthylphényl)-N-4-[4-(pipéridinométhyl)phényl]-cyclohexylthiocarbamate et
(17) trans-N-4-[4-(diméthylaminométhyl)phényl]cyclohexyl-O-(4-fluorophényl)-N-méthylcarbamate,
leurs mélanges et leurs sels.

5. Sels physiologiquement acceptables des composés selon au moins l'une des revendications 1 à 4 avec des acides inorganiques ou organiques.

6. Médicament contenant un composé selon au moins l'une des revendications 1 à 4 ou un sel physiologiquement acceptable selon la revendication 5 outre éventuellement un ou plusieurs supports et/ou diluants inertes.

7. Médicament selon la revendication 6 convenant pour le traitement de maladies dans lesquelles la biosynthèse du cholestérol joue un rôle.

8. Médicament selon la revendication 7 contenant un composé selon au moins l'une des revendications 1 à 4 ou un sel physiologiquement acceptable selon la revendication 5 en combinaison avec un ou plusieurs autres principes actifs à activité abaissant le cholestérol ou les lipides.

9. Médicament selon la revendication 8 **caractérisé en ce que** les autres principes actifs sont choisis parmi
les résines liant les acides biliaires,
les composés qui inhibent la résorption du cholestérol,
les composés qui interviennent dans la biosynthèse du cholestérol par le biais d'un autre mécanisme que l'inhibition de la 2,3-époxysqualène-lanostérol-cyclase,
les fibrates, l'acide nicotinique, leurs dérivés et analogues, ainsi que le probucol.

10. Médicament selon la revendication 7, 8 ou 9 convenant pour le traitement ou la prophylaxie de l'hypercholestérolémie, de l'hyperlipoprotéinémie, de l'hypertriglycéridémie et des modifications vasculaires athérosclérotiques qui en résultent avec leurs maladies consécutives comme la maladie cardiaque coronarienne, l'ischémie cérébrale, la claudication intermittente ou la gangrène, pour le traitement de maladies qui sont en liaison avec la prolifération cellulaire excessive, pour la prophylaxie et le traitement des affections de type calculs biliaires ou pour le traitement des mycoses.

11. Utilisation d'un composé selon au moins l'une des revendications 1 à 5 pour la préparation d'un médicament selon l'une des revendications 7 à 10.

12. Aliment pour poules pondeuses contenant un composé selon au moins l'une des revendications 1 à 4 ou un sel physiologiquement acceptable selon la revendication 5.

13. Utilisation d'un composé selon au moins l'une des revendications 1 à 5 pour la préparation d'un aliment pour poules pondeuses pour la production d'oeufs pauvres en cholestérol.

14. Procédé de préparation d'un médicament selon la revendication 6 **caractérisé en ce que**, par voie non chimique, un composé selon au moins l'une des revendications 1 à 5 est incorporé dans un ou plusieurs supports et/ou diluants inertes.

15. Procédé de préparation des composés selon au moins l'une des revendications 1 à 5 **caractérisé en ce que**
a) un composé de formule générale 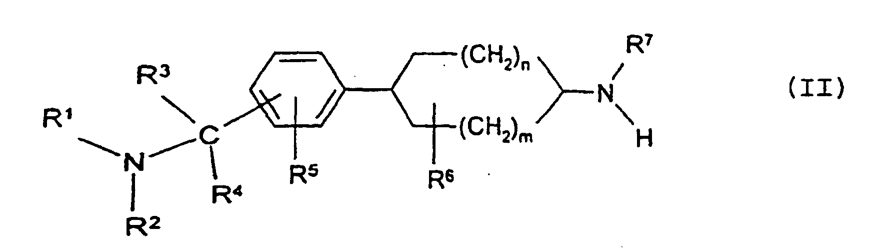 où
m, n et R¹ à R⁷ sont définis comme dans les revendications 1 à 4 est mis à réagir avec un composé de formule générale 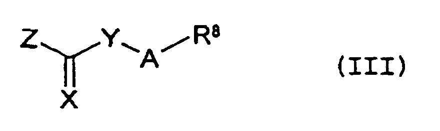 où
A, X, Y et R⁸ sont définis comme dans les revendications 1 à 4 et Z signifie un groupe partant,
puis les groupes protecteurs éventuellement utilisés sont clivés ou
b) pour la préparation de composés de formule générale (I) où X et Y signifient chacun un atome de soufre et m, n, A et R¹ à R⁸ sont définis comme dans les revendications 1 à 4 avec la condition que R⁸ ne représente pas un groupe phényle ou naphtyle éventuellement substitué, au cas où A signifie une simple liaison, un composé de formule générale (II), où m, n et R¹ à R⁷ sont définis comme dans les revendications 1 à 4 est mis à réagir avec un le sulfure de carbone puis avec un agent d'alkylation de formule générale 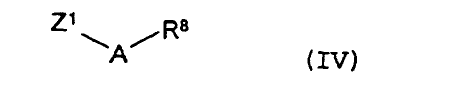 où
A et R⁸ sont définis comme dans les revendications 1 à 4 avec la condition que R⁸ ne représente pas un groupe phényle ou naphtyle éventuellement substitué, au cas où A signifie une simple liaison, et Z¹ signifie un groupe partant, puis les groupes protecteurs éventuellement utilisés sont clivés et
si on le souhaite un mélange des isomères géométriques d'un composé de formule générale I ainsi obtenu est résolu en ses énantiomères et diastéréoisomères ou un composé de formule générale I ainsi obtenu est converti en son sel avec un acide inorganique ou organique, en particulier en ses sels physiologiquement acceptables.
